# EUROPÄISCHE PATENTSCHRIFT

(11) **EP 2 887 887 B1**
(45) Veröffentlichungstag und Bekanntmachung des Hinweises auf die Patenterteilung: **29.12.2021**
(21) Anmeldenummer: 13766232.6
(22) Anmeldetag: 22.08.2013
(51) Int. Cl.: A61B 17/12

(54) **IMPLANTAT**
IMPLANT
IMPLANT

(30) Priorität: 22.08.2012 DE 102012016555; 11.01.2013 DE 102013000288; 16.04.2013 DE 102013006503
(43) Veröffentlichungstag der Anmeldung: 01.07.2015
(73) Patentinhaber: Phenox GmbH, 44801 Bochum (DE)
(72) Erfinder: MONSTADT, Hermann, 44797 Bochum (DE); HANNES, Ralf, 44137 Dortmund (DE); HERKLOTZ, Dennis, 42389 Wuppertal (DE)
(74) Vertreter: Schneiders, Josef
(86) Internationale Anmeldenummer: PCT/EP2013/067439
(87) Internationale Veröffentlichungsnummer: WO 2014/029835

(56) Entgegenhaltungen:
- WO-A1-02/00139
- WO-A1-2012/113554
- WO-A2-2006/052322
- DE-A1-102008 028 308
- US-A1- 2007 198 075

## Beschreibung

Die Erfindung betrifft ein Implantat zum Einsatz bei der Okkludierung von Aneurysmen in Gefäßverzweigungen, insbesondere von Bifurkationsaneurysmen. Ein solches Implantat wird mit Hilfe eines Katheters und Führungsdrahts an den Implantationsort gebracht und dort dauerhaft implantiert. Entsprechend betrifft die Erfindung auch ein solches Implantat, implantationsfertig angekoppelt an einen Führungsdraht. Darüber hinaus betrifft die Erfindung ein Verfahren zur Einbringung des Implantats.

Arteriovenöse Fehlbildungen können in einem Patienten zu erheblichen Beeinträchtigungen und Gefährdungen bis hin zum Tode führen. Dies gilt insbesondere auch für Aneurysmen, insbesondere dann, wenn sie im zerebralen Bereich auftreten. In der Regel versucht man derartige Fehlbildungen durch Implantate zu verschließen. Solche Implantate werden in der Regel auf endovaskulärem Weg mit Hilfe von Kathetern gesetzt.

Insbesondere bei zerebralen Aneurysmen hat sich die Implantierung von Platinspiralen bewährt, die das Aneurysma mehr oder weniger vollständig ausfüllen, den Bluteinstrom weitgehend blockieren und dazu führen, dass sich ein lokaler Thrombus ausbildet, der das Aneurysma ausfüllt und letztlich verschließt. Diese Behandlungsmethode ist allerdings nur bei Aneurysmen geeignet, die über einen relativ engen Zugang zum Gefäßsystem verfügen, sogenannten Beerenaneurysmen. Bei Aussackungen von Blutgefäßen, die über einen weiten Zugang zum Gefäß verfügen, drohen die implantierten Spiralen wieder ausgeschwemmt zu werden. Diese können in andere Bereiche des Gefäßsystems gelangen und dort Schäden herbeiführen.

In solchen Fällen wurde bereits vorgeschlagen, eine Art Stent zu setzen, der die Öffnung des Aneurysmas "vergittert" und dadurch die Ausschwemmung der Okklusionsspiralen verhindert. Derartige Stents, die über eine relativ weitmaschige Wandung verfügen, werden bereits bei einigen Formen von Aneurysmen eingesetzt.

Dokumente US 2007/198075 A1, DE 10 2008 028308 A1 und WO 2006/052322 A2 offenbaren vaskuläre Implantate zur Behandlung von Aneurysmen im Bereich von Gefäßverzweigungen.

Dokument WO2012113554 A gehört zum Stand der Technik gemäß Art. 54 (3) EPÜ und betrifft ein Implantat zum Einsatz bei der Okkludierung von Aneurysmen im Bereich von Gefäßverzweigungen, insbesondere Bifurkationsaneurysmen, mit einer Maschenstruktur, welches - von proximal nach distal - die Abschnitte (a) bis (d) aufweist: (a) einen sich verjüngenden proximalen Abschnitt, in dem die Maschenstruktur zu einem oder mehreren Kupplungselementen zusammengeführt ist, (b) einen Fixierabschnitt, mit dem das Implantat an einer Gefäßwand abstützbar ist, (c) einen durchlässigen Abschnitt für den Bereich der Gefäßbifurkation und (d) einen distalen Abschnitt, in dem das Implantat gegenüber den Abschnitt (b) erweitert ist und der zur Platzierung im Aneurysma bestimmt ist, wobei im Bereich der Abschnitte (c) oder (d) eine Trennzone angeordnet ist. Der Abschnitt (d) besteht wesentlich aus mit einer dehnfähigen Membran versehene Drahtschlaufen.

Gefäßverzweigungen, insbesondere Gefäßbifurkationen, sind ein relativ häufiges Phänomen. Das durch eine Arterie im Bereich einer Gabelung auf die Stirnwand aufprallende Blut führt - im Fall einer Schwächung der Gefäßwandung - schnell zu einer Aussackung, die sich dann rasch erweitert. Solche Bifurkationsaneurysmen haben häufig einen weiten Hals, der eine Therapie nur mit Okklusionsspiralen unmöglich macht.

Gleichzeitig fehlt es an Stentstrukturen, die geeignet sind, im Bereich einer Gefäßverzweigung eine "Vergitterung" des Aneurysmaeingangs herbeizuführen. Unter diesem Gesichtspunkt ist es Aufgabe der Erfindung, ein Implantat bereitzustellen, das geeignet ist, im Bereich insbesondere von Bifurkationsaneurysmen eingesetzt zu werden und dort den Eingang eines Aneurysmas zu "vergittern". Mit anschließend eingebrachten Okklusionsspiralen kann dann das Aneurysma stillgelegt werden.

Eine solche "Vergitterung" ist auch im Sinne einer Beeinflussung des Blutstroms denkbar, um die Anzahl der Okklusionsspiralen zu reduzieren oder auf null zu setzen.

Diese Aufgabe wird mit einem Implantat mit einer Maschenstruktur gelöst, welches - von proximal nach distal - die Abschnitte (b) bis (d) aufweist:
einen Fixierabschnitt (b), mit dem das Implantat an einer Gefäßwand abstützbar ist,
einen durchlässigen Abschnitt (c) für den Bereich der Gefäßverzweigung und
einen distalen Abschnitt (d), in dem das Implantat gegenüber dem Abschnitt (b) radial erweitert ist und der zur Platzierung im Aneurysma bestimmt ist,
wobei im Bereich der Abschnitte (c) oder (d) eine Trennzone angeordnet ist, die den Hals des Aneurysmas zumindest teilweise verschließt, der distale Abschnitt (d) eine Mehrzahl von mit dem Abschnitt (c) verbundenen Schlaufen aufweist und die Schlaufen zur Längsachse des Implantats einen Winkel zwischen -45° und +175° ausbilden, wobei ein positiver Winkel für nach radial außen und ein negativer Winkel für nach radial innen weisende Schlaufen steht und wobei die Trennzone eine oder mehrere Membranen aufweist, die das Innere der Schlaufen ausfüllen und Zwischenräume zwischen den Schlaufen überspannen.

Die Begriffe "proximal" und "distal" sind so zu verstehen, dass sie zum Führungsdraht und somit zum Katheter und behandelnden Arzt weisende Teile des Implantats bezeichnen (proximal) bzw. vom Führungsdraht oder behandelnden Arzt weg weisende Teile (distal). Proximal ist damit führungsdrahtseitig und distal führungsdrahtabgewandt. Der Begriff "axial" bezieht sich auf die von proximal nach distal verlaufende Längsachse des Implantats, der Begriff "radial" auf hierzu senkrechte Ebenen.

Bei dem erfindungsgemäßen Implantat handelt es sich um ein Implantat mit einer Maschenstruktur, die aus einem Geflecht einzelner Drähte bestehen kann, eine aus einem Rohr geschnittene Maschenstruktur aufweisen kann oder eine Kombination von beidem ist. Insoweit handelt es sich bei dem Implantat weitgehend um einen Stent oder ein stentähnliches Gebilde, das sich durch die besondere Art seines Einsatzes und Aufbaus auszeichnet. Im Falle der Flechtung aus einzelnen Drähten wird für die Abschnitte (b) und (c) eine Zahl von 4 bis 24 Drähten bevorzugt.

Das erfindungsgemäße Implantat ist in mindestens drei, vorzugsweise vier Abschnitte gegliedert, nämlich die Abschnitte (a) bis (d), von proximal nach distal gesehen, wobei der Abschnitt (a) optional ist. Die Abschnitte (b) und (c) können auch gleich aufgebaut sein und sich lediglich hinsichtlich der Lage im Gefäß nach der Implantierung unterscheiden.

Der Abschnitt (a) ist ein sich verjüngender proximaler Abschnitt, in dem die Maschenstruktur zu einem oder mehreren Kupplungselementen zusammengeführt ist. Die Kupplungselemente befinden sich vorzugsweise an der Peripherie, d. h. kommen im implantierten Zustand, wenn das Implantat in seiner expandierten Form vorliegt, an der Gefäßwandung zu liegen, und dienen der Verbindung mit einer Einführhilfe, insbesondere einem Führungsdraht. Eine zentrierte Anordnung ist auch aus applikationstechnischen Gründen nicht sinnvoll; die periphere Anordnung des oder der Kupplungselemente erleichtert bei Fehlplatzierungen das Rückziehen des Implantats in den Platzierungskatheter. Bevorzugt sind Ausführungsformen mit einem oder zwei Kupplungselementen. Vorzugsweise handelt es sich bei den Kupplungselementen um Kupplungsdrähte.

Die Kupplungselemente, insbesondere die Kupplungsdrähte, bzw. das proximale Ende des Implantats (ohne Einführhilfe) können zur Längsachse des Implantats einen Winkel zwischen 0° und +60° ausbilden, wobei ein positiver Winkel für ein nach außen weisendes proximales Ende steht. Bevorzugt ist ein Bereich zwischen +10° und +30°, wobei der optimale Winkel von der Gestalt des Gefäßes abhängt. Ein solcher positiver Winkel erleichtert die optimale Expansion des Implantats und das Anlegen des proximalen Endes an das Trägergefäß; das Hineinragen des proximalen Endes in das Gefäßlumen, das den Blutfluss oder das Einführen eines weiteren Mikrokatheters stören könnte, wird wirkungsvoll verhindert. Vorzugsweise ist das proximale Ende des Implantats atraumatisch ausgebildet, um eine Verletzung der Gefäßwand auszuschließen. Die Ausbildung des Winkels ist erfindungsgemäß so zu verstehen, dass der Winkel nicht im unimplantierten Zustand vorliegen muss, es kommt vielmehr darauf an, dass das proximale Ende des Implantats einen solchen Winkel nach Implantation ausbildet, d. h. es ist ausreichend, dem Implantat eine entsprechende Verformung nach Implantation aufzuprägen. Hier bietet sich insbesondere die Verwendung von Formgedächtnismaterialien an.

Abschnitt (b) ist ein Fixierabschnitt, mit dem sich das Implantat an der Gefäßwand des Blut heranführenden Gefäßes abstützt. In diesem Bereich ist das Gefäß nicht geschädigt und geeignet, mit einer Stentwandung beaufschlagt zu werden. Bei selbstexpandierenden Implantaten legt sich der Abschnitt (b) nach der Freisetzung aus dem Katheter selbständig an die Gefäßwandung an, bei ballonplatzierbaren Implantaten wird das Implantat in diesem Bereich durch einen Platzierungsballon aufgeweitet und gegen die Gefäßwand gepresst.

Abschnitt (c) ist ein durchlässiger Abschnitt, der insbesondere eine größere Maschenweite als Abschnitt (b) aufweisen kann und der in den Bereich der eigentlichen Gefäßbifurkation gesetzt wird. Eine größere Maschenweite erlaubt einen mehr oder weniger ungehemmten Blutfluss durch die Maschen hindurch in die abführenden Gefäßzweige. Es ist jedoch nicht in jedem Fall erforderlich, im Abschnitt (c) eine größere Maschenweite vorzusehen; sofern dies nicht der Fall ist, können die Abschnitt (b) und (c) auch weitgehend oder vollständig identisch aufgebaut sein und sich nur hinsichtlich der Lage bei Einbringung in das Gefäßsystem unterscheiden.

Der distale Abschnitt (d) ist gegenüber dem Abschnitt (b) und zumeist auch gegenüber dem Abschnitt (c) radial nach außen erweitert. Er dient der Platzierung im Aneurysma selbst, an dessen sich erweiternde Wandung er sich anpassen soll.

Im Bereich der Abschnitte (c) und (d), insbesondere zwischen den Abschnitten (c) und (d) ist eine Trennzone angeordnet, die den Aneurysmenhals abdichtet. Die Trennzone dient insbesondere der Rückhaltung von in das Bifurkationsaneurysma eingebrachten Okklusionsmitteln. Im Falle einer hinreichend dichten Trennzone, die den Aneurysmenhals in ausreichendem Maße abdichtet, ist es aber auch denkbar, auf zusätzliche Okklusionsmittel wie Coils zu verzichten. Wichtig ist, dass letztlich im Aneurysma eine Blutgerinnung einsetzt. In jedem Fall steht die Trennzone in das Lumen des Implantats orthogonal zur Längsachse hinein. Die Abdeckungsrate des Aneurysmenhalses liegt zwischen 5 und 100 %, wobei Werte zwischen 30 und 60 % bevorzugt sind. Auf der einen Seite muss die Oberflächenabdeckung hinreichend groß sein, um in das Aneurysma eingebrachte Okklusionsmittel daran zu hindern, aus dem Aneurysma zu entweichen, oder durch ausreichendes Material eine dichte Oberfläche zu schaffen, auf der anderen Seite muss eine hinreichend große Flexibilität des Implantats erhalten bleiben, um es im Bereich des Bifurkationsaneurysmas einbringen zu können.

Denkbar sind auch Ausführungsformen des Implantats, die keine Trennzone aufweisen, diese sind jedoch nicht als erfindungsgemäß aufzufassen. Ein solches Implantat kann eingesetzt werden, wenn mehr als ein Implantat in den Bereich des Bifurkationsaneurysmas eingebracht werden soll, insbesondere zwei Implantate. Dies kann von Vorteil sein, wenn das Aneurysma sehr unregelmäßig aufgebaut ist und in einem Teilbereich des Aneurysmas ein Verschluss herbeigeführt werden soll, in einem anderen Teilbereich hingegen der Blutfluss aufrecht erhalten werden muss, da sich Aneurysma und abgehendes Gefäß überschneiden. Bei dieser Variante bringt man zunächst ein Implantat ohne Trennzone ein, das im Übrigen vollständig dem beschriebenen Implantat entspricht. In einem zweiten Schritt wird durch das erste Implantat hindurch ein weiteres Implantat mit Trennzone eingebracht, um den Verschluss des Aneurysmas im gewünschten Umfang zu gewährleisten. Durch Einbringung von zwei aufeinander abgestimmten Implantaten kann den besonderen Anforderungen des Aneurysmas Rechnung getragen werden, beispielsweise wenn diese unterschiedliche distale Abschnitte (d) oder durchlässige Abschnitte (c) aufweisen.

Die Erweiterung des Abschnitts (d) wird durch mit dem Abschnitt (c) verbundene Schlaufen gebildet. Die Erweiterung weist wenigstens zwei Schlaufen auf, insbesondere drei Schlaufen oder mehr. Typischerweise beträgt die Zahl der Schlaufen 2 bis 24, bevorzugt sind 2 bis 6. Bei den Schlaufen kann es sich um entsprechend geformte Drahtelemente handeln, sie können aber auch, soweit das Implantat aus einem Rohr geschnitten ist, entsprechend durch Laserschneiden des gleichen Rohrs erzeugt sein. Bei den Schlaufen handelt es sich vorzugsweise um vom Abschnitt (c) ausgehende, eine Schleife ausbildende und zurück verlaufende Drahtelemente, wobei grundsätzlich beliebig komplexe Formen für die Schlaufen denkbar sind. Insbesondere kann es sich auch um dreidimensionale Objekte handeln, je nachdem, wie die Schlaufen verlaufen. Schlaufen sind vorteilhaft, da sie weitgehend atraumatisch sind und die empfindliche Gefäßwand des Aneurysmas nicht verletzen. Die Erweiterung kann beispielsweise trompetenförmig, korbförmig oder in Form eines Geflechts vorliegen.

Der Winkel, den die Schlaufen zur Längsachse des Implantats im implantierten Zustand ausbilden, liegt zwischen -45° und +175°, wobei ein positiver Winkel für nach radial außen und ein negativer Winkel für nach radial innen weisende Schlaufen steht. Im Falle von verhältnismäßig regelmäßigen Bifurkationsaneurysmen liegt der Winkel bevorzugt bei +45° bis +90°, teilweise treten jedoch auch Aneurysmen auf, die eine unregelmäßige Form aufweisen, insbesondere stark asymmetrisch sind. In solchen Fällen kann es sinnvoll sein, stark abweichende Winkel der Schlaufen zum Einsatz zu bringen. Beispielsweise kann es sinnvoll sein, den Winkel sehr groß zu wählen, wenn die Wandung in einem Bereich des Aneurysmas stark in Richtung des zuführenden Gefäßes ausgewölbt ist. In solchen Fällen sind Winkel > 90° sinnvoll. In anderen Fällen kann es zweckmäßig sein, einen Teil der Schlaufen nach innen weisen zu lassen, d. h. negative Winkel zu wählen, um sich an die Wandung des Aneurysmas anzupassen. Die Winkel, die die einzelnen Schlaufen ausbilden, können variieren, beispielsweise kann es bei einem asymmetrischen Aneurysma sinnvoll sein, einige Schlaufen mit Winkeln > 90° vorzusehen, während andere Schlaufen herkömmliche Winkel im Bereich zwischen 45° und 90° aufweisen. Wichtig ist, dass die genannten Winkel nach Implantierung ausgebildet werden, auch ein Implantat, bei dem im Zustand vor der Implantierung, etwa durch äußere Zwänge, sich die angegebenen Winkel noch nicht ausgebildet haben, ist daher als erfindungsgemäß anzusehen.

Winkel, die die Schlaufen zur Längsachse des Implantats ausbilden, können z. B. zwischen 45° und 90°, -45° und 0°, 90° und 135° oder 135° und 175° liegen.

Die Schlaufen im Abschnitt (d) können Fortsetzungen der den übrigen Implantatkörper ausbildenden Drähte oder Stege sein, es kann sich aber auch um gesonderte Drahtfilamente handeln, die im distalen Bereich des übrigen Implantatkörpers, d. h. am distalen Ende des Abschnitts (c) festgelegt sind, beispielsweise durch Laserverschweißen. Dabei kann jede Schlaufe des Abschnitts (d) an einem oder mehreren Verbindungspunkten mit dem weiteren Implantatkörper verbunden sein, insbesondere können auch nur ein oder zwei Verbindungspunkte pro Schlaufe vorgesehen sein.

Gemäß einer alternativen, nicht erfindungsgemäßen Ausführungsform wird ein Implantat zum Einsatz bei der Okkludierung von Aneurysmen im Bereich von Gefäßverzweigungen, insbesondere Bifurkationsaneurysmen, mit einer Maschenstruktur zur Verfügung gestellt, welches - von proximal nach distal - die Abschnitte (b) bis (d) aufweist:
einen Fixierabschnitt (b), mit dem das Implantat an einer Gefäßwand abstützbar ist,
einen durchlässigen Abschnitt (c) für den Bereich der Gefäßverzweigung und
einen distalen Abschnitt (d), in dem das Implantat gegenüber dem Abschnitt (b) radial erweitert ist und der zur Platzierung im Aneurysma bestimmt ist, wobei im Bereich der Abschnitte (c) oder (d) eine Trennzone angeordnet ist, die den Hals des Aneurysmas zumindest teilweise verschließt, und der distale Abschnitt (d) kugelförmig, pilzförmig, ankerförmig oder ellipsoidförmig erweitert ist. Vorzugsweise ist der distale Abschnitt (d) nicht zentral am Abschnitt (c) angebunden, sondern peripher. Abgesehen von der abweichenden Ausbildung des distalen Abschnitts (d) gilt für alle weiteren Aspekte des Implantats das zur Ausführungsform nach Anspruch 1 gesagte.

Bei den genannten Formen handelt es sich um Alternativen, durch die ebenfalls ein radial erweiterter Abschnitt (d) ausgebildet werden kann. Ein kugelförmiger Abschnitt (d) kann sich beispielsweise gut an die Innenwandungen des Aneurysmas anschmiegen, da ein regelmäßiges Bifurkationsaneurysma häufig im Wesentlichen die Form einer Kugel aufweist. Dabei werden als Kugelform nicht nur exakte Kugeln entsprechend der geometrischen Definition verstanden, vielmehr werden auch hiervon abweichende, runde, dreidimensionale Formen erfindungsgemäß als Kugeln angesehen. In einigen Fällen ähnelt die Form des Abschnitts (d) auch einem Ellipsoid, wobei auch hier gilt, dass das Vorliegen eines exakten Rotationsellipsoids nicht erforderlich ist, um als ellipsoidförmig zu gelten. Weitere Möglichkeiten sind pilz- oder ankerförmige Abschnitte (d), die sich insbesondere bei unregelmäßigen Aneurysmen eignen, beispielsweise wenn die Wandung in einem Bereich des Aneurysmas stark in Richtung des zuführenden Gefäßes ausgewölbt ist. Dies wird dadurch gewährleistet, dass bei einer Pilz- oder Ankerform einige Bereiche des Abschnitts (d) in proximaler Richtung verlaufen. Dabei sei klargestellt, dass ein pilz- oder ankerförmiger Abschnitt selbst ebenfalls asymmetrisch sein, beispielsweise nur auf einer Seite in proximaler Richtung verlaufende Bereiche aufweisen kann. Sofern der Abschnitt (d) hinreichend oberflächendicht ist, kann er selbst die Trennzone ausbilden, auf gesonderte Vorrichtungen kann somit ggf. verzichtet werden. Der distale Abschnitt (d) kann lasergeschnitten oder auch geflochten sein, wobei bevorzugt 8 bis 128 Drähte zum Einsatz kommen.

Die erfindungsgemäßen Implantate können aus herkömmlichen Stentmaterialien gefertigt sein, beispielsweise aus medizinischem Stahl oder Kobaltchromlegierungen bestehen, aber insbesondere aus Formgedächtnismaterial, etwa Nitinol oder ternären Nickeltitanlegierungen.

Wie schon angesprochen, ist ein erfindungsgemäßes Implantat vorzugsweise zumindest partiell aus einem Rohr geschnitten, insbesondere aus einem Rohr aus einer Formgedächtnislegierung. Auch die Trennzone kann aus dem Rohr geschnitten sein.

Die im erfindungsgemäßen Implantat vorgesehene Trennzone verläuft insbesondere zwischen den Abschnitten (c) und (d). In diesem Zusammenhang ist festzustellen, dass der Abschnitt (c) zumindest in seinem distalen Ende gegenüber dem Abschnitt (b) erweitert sein kann, was dann hilfreich ist, wenn das Bifurkationsaneurysma bereits Teile der abgehenden Blutgefäße eingenommen hat. In diesem Fall muss der Eingangsteil des Aneurysmas für den abzweigenden Blutstrom freigehalten werden, so dass die Trennzone im Aneurysma selbst verläuft. Der bereits erweiterte Bereich des Abschnitts (c) geht dann, ggf. unter weiterer Aufweitung, in den Abschnitt (d) über. Auch hier liegt die Trennzone zwischen den Abschnitten (c) und (d). Bei einer sehr flachen Ausgestaltung des Abschnitts (d) kann die Trennzone auch mit dem Abschnitt (d) zusammenfallen.

Die Trennzone kann einerseits durch das Einziehen von Fasern, Fäden, dünnen Drähten, einer Membran oder dergleichen Trennelemente ausgebildet werden, kann aber auch integraler Teil des Implantats in dem Sinne sein, dass es sich um aus dem Ausgangsrohr herausgeschnittene und entsprechend umgeformte Trennelemente oder um aus einem Drahtgeflecht gebildete Trennelemente, etwa Schlaufen oder Stege, handelt. Im Falle von Schlaufen oder Stegen weisen diese radial nach innen in das Lumen des Implantats, im Gegensatz zu Schlaufen des distalen Abschnitts (d), die zumindest größtenteils nach außen weisen. Damit die nach innen weisenden Schlaufen/Stege sich nicht gegenseitig behindern, kann es sinnvoll sein, diese asymmetrisch auszugestalten. Die Zahl kann variieren, abhängig von der Struktur des Implantats und der Anzahl der Waben.

Die die Trennzone ausbildenden Fäden können aus einem Polymermaterial hergestellt sein, beispielsweise einem Polyamid wie Nylon (Polyhexamethylenadipinsäureamid). Ebenso möglich ist die Herstellung aus Metall, wobei Formgedächtnislegierungen bevorzugt sind, insbesondere Nickel-Titan-Legierungen wie Nitinol.

Eine weitere Möglichkeit besteht darin, in der Trennzone eine Membran vorzusehen, die weitgehend oder vollständig undurchlässig für Blut ist und das Aneurysma auf diese Weise vom Blutstrom abkoppelt. Sofern eine weitgehend vollständige Abkopplung vom Blutstrom erreicht wird, ist eine Einbringung von Okklusionsmitteln in das Aneurysma u. U. entbehrlich, d. h. die Trennzone dient in diesem Fall nicht zum Zurückhalten von Okklusionsmitteln. Die Membran kann auf ein Geflecht aus Fäden oder Drähten aufgespannt sein, bspw. können Fäden oder Drähte eine Struktur bilden, über oder auf die die Membran aufgespannt ist. Zusätzlich sind weitere, z. B. kreuzförmig verlaufende Fäden/Drähte denkbar, die ein Fadenkreuz ausbilden. Fäden oder Drähte sind jedoch nicht unbedingt erforderlich, auch ein Überspannen der Trennzone ohne zusätzliche Fäden/Drähte ist möglich.

Es kann jedoch von Vorteil sein, zusätzlich Okklusionsmittel in das Aneurysma einzubringen. Aus diesem Grund kann es sinnvoll sein, für die Trennzone eine Membran mit einer oder mehreren Ausnehmungen zu verwenden, so dass durch die Ausnehmung ein Einbringen von Okklusionsmitteln, insbesondere Coils möglich ist. Die Ausnehmung sollte eine Größe aufweisen, dass ein Katheter durch die Ausnehmung in den Bereich des Aneurysmas vorgeschoben werden kann, wobei durch den Katheter die Okklusionsmittel eingebracht werden. Andererseits sollte die Ausnehmung den Aneurysmenhals so weit abdecken, dass die Okklusionsmittel das Aneurysma nicht unkontrolliert verlassen können, wobei ggf. die Trennzone überspannende Fäden/Drähte eine zusätzliche Rückhaltefunktion erfüllen. Selbstverständlich dürfen in diesem Fall die Fäden bzw. Drähte nur so dicht verlaufen, dass ein Hindurchführen eines Katheters und ein Einbringen von Okklusionsmitteln weiterhin möglich sind.

Um Okklusionsmittel in das Aneurysma einzubringen, kann die die Trennzone überspannende Membran auch partiell durchstoßbar ausgebildet werden, wobei zum Durchstoßen typischerweise ein Mikrokatheter oder ein Führungsdraht verwendet wird. Durch die so geschaffene Öffnung wird sodann ein Mikrokatheter geführt, durch den die Okklusionsmittel eingeschoben werden. Die Membran sollte so beschaffen sein, dass sie auch nach Durchstoßen partiell erhalten bleibt, so dass die Okklusionsmittel weiterhin durch die Membran am Wiederaustritt gehindert werden. Beispielsweise können in der Trennzone vorgesehene Fäden oder Drähte, die in der Form eines Fadenkreuzes aufgespannt sein können, dafür sorgen, dass lediglich ein Segment der Membran beim Durchstoßen eine Öffnung ausbildet, während die übrigen Segmente von der Membran abgedeckt bleiben, da die Randbereiche der Membran von den Fäden/Drähte stabilisiert und vor einem Einreißen geschützt werden. Bei der die Trennzone überspannenden Membran kann es sich um eine einzelne Membran handeln, die nur partiell durchstoßen wird, oder auch um mehrere kleine Membranen.

Im Inneren der den Abschnitt (d) bildenden (Draht)schlaufen sind Membranen vorgesehen. Sofern sowohl in der Trennzone als auch im Inneren der Schlaufen Membranen vorgesehen sind, erleichtert dies die Fixierung der Membran.

Die Membran muss nicht auf die Trennzone und das Innere der Schlaufen beschränkt sein, sondern kann insgesamt Trennzone und Schlaufen überspannen, wobei die Schlaufen zur Fixierung der Membran dienen können. Membranen sind auch in den Zwischenräumen zwischen den Schlaufen vorgesehen.

Zur Abgrenzung und Verstärkung der Membran können auch zwischen den einzelnen Schlaufen gespannte Fäden dienen, d. h. die Membranen werden zumindest teilweise seitlich von einem oder mehreren Fäden begrenzt, die die Schlaufen miteinander verbinden. Dabei muss die Begrenzung der jeweiligen Membran nicht in jeder Richtung über einen Faden erfolgen, hierzu können auch teilweise die Schlaufen selbst dienen. Beispielsweise kann der äußere Rand der Membran, der häufig auch weiter distal liegt, durch Fäden, der innere Rand durch Schlaufen umrandet sein. Im Vergleich zu einer Membran ohne seitliche Begrenzung wird insbesondere ein zusätzlicher Schutz der Membran gegen Beschädigungen und Risse erreicht. Die Fäden werden bevorzugt aus einem Polyamid wie Nylon gefertigt.

Ein Vorteil der Verwendung einer Membran im Bereich der Trennzone ist darin zu sehen, dass diese sich bei der Platzierung des Implantats im Katheter eng nach distal oder proximal zusammenlegt, so dass ein Implantat mit im expandierten Zustand weitgehend dichter Trennzone zur Verfügung gestellt wird, das sich im kontrahierten Zustand auch durch enge Blutgefäße gut hindurchführen lässt. Im Übrigen bleibt der Aufbau des Implantats im Vergleich zu einem Implantat ohne Trennzone weitgehend unverändert.

Die Membran kann aus einem Polymermaterial wie Polytetrafluorethylen, Polyester, Polyamiden, Polyurethanen oder Polyolefinen hergestellt sein. Besonders bevorzugt sind Polycarbonaturethane. Wünschenswert ist insbesondere eine integrale Verbindung der Membran mit den die Trennzone ausbildenden Fäden oder Drähten. Diese kann durch eine Tauch- oder Sprühbeschichtung der Fäden/Drähte herbeigeführt werden.

Vorzugsweise wird die Membran durch Elektrospinnen erzeugt. Hierbei werden Fibrillen bzw. Fasern aus einer Polymerlösung mit Hilfe von elektrischem Strom auf einem Substrat abgeschieden. Bei der Abscheidung verkleben die Fibrillen zu einem Vlies. In der Regel haben die Fibrillen einen Durchmesser von 100 bis 3000 nm. Durch Elektrospinnen gewonnene Membranen sind sehr gleichmäßig ausgebildet und können eine Grundstruktur aus Fäden oder Drähten in sich einzuschließen. Die Membran ist zäh und mechanisch belastbar und kann mechanisch durchstoßen werden, ohne dass die Öffnung zum Ansatzpunkt für weitergehende Risse wird. Die Dicke der Fibrillen wie auch der Grad der Porosität kann durch Auswahl der Verfahrensparameter gesteuert werden. Im Zusammenhang mit der Schaffung der Membran und den dafür geeigneten Materialien wird insbesondere auf die WO 2008/049386, die DE 28 06 030 A1 und die darin genannte Literatur hingewiesen.

Vorteilhaft ist auch ein Implantat, bei dem die Trennzone durch eine an der Innenseite des Implantats anliegende Membran gebildet wird, wobei diese Membran wiederum mit weiteren äußeren Membranabschnitten fest verbunden ist, die die einzelnen Schlaufen ausfüllen. Eine solche Membranstruktur lässt sich über Elektrospinnen erzeugen. Die innere und äußere Membranschicht sind in diesem Fall teilweise verbunden; dort, wo die innere Membranschicht keine Verbindung mit der äußeren Membranschicht aufweist, zieht sie sich ähnlich einem Nylonstrumpf zusammen und bildet somit eine passierbare Trennzone aus.

Statt durch Elektrospinnen kann die Membran auch über einen Tauchprozess hergestellt werden.

Die Membran muss nicht in jedem Fall in einer Ebene orthogonal zur Längsachse des Implantats verlaufen, sondern kann auch eine Ausrichtung in Richtung proximal aufweisen. In diesem Fall ist die Membran zwar in ihrem Randbereich im Umfang des Implantats fixiert, der mittlere Bereich der Membran erstreckt sich aber in proximaler Richtung. Es ergibt sich somit insgesamt eine Kegel- oder Pyramidenform, wobei die Basis des Kegels/der Pyramide orthogonal zur Längsachse orientiert und die Membran im Randbereich mit dem Implantat verbunden ist, während der Scheitel des Kegels/der Pyramide weiter proximal liegt. Der Blutstrom wird, wenn er auf die Membran trifft, auf diese Weise geteilt und seitwärts abgelenkt, so dass der Blutstrom in das Aneurysma weitgehend zum Erliegen kommt.

Auch im Falle einer Kegel-/Pyramidenform der die Trennzone ausbildenden Membran kann diese eine oder mehrere Ausnehmungen aufweisen, damit weiterhin nach Platzierung des Implantats durch die Ausnehmung hindurch Okklusionsmittel in das Aneurysma eingebracht werden können.

Um die Kegel-/Pyramidenform der Membran dauerhaft zu erhalten, sollte die Membran auf einer Gerüststruktur aus Fäden oder Drähten fixiert sein, wobei es sich prinzipiell auch um Stege handeln kann, die aus der das Implantat ausbildenden Struktur z. B. mit Hilfe eines Lasers herausgeschnitten sind. Dabei sollte darauf geachtet werden, dass die Fäden/Drähte eine hinreichende Steifigkeit aufweisen, um eine Umorientierung oder Umstülpung der Membran aufgrund des Blutdrucks zu verhindern. Ggf. müssen hierfür zusätzliche Fäden oder Drähte eingebracht werden.

Eine Möglichkeit besteht darin, ein Fadenkreuz aus zwei relativ langen Einzelfäden zu erzeugen, an dem die Membran fixiert ist, wobei aufgrund der Länge der Einzelfäden die Membran zunächst nicht gespannt ist. Ein oder mehrere Fäden können darüber hinaus an einer weiter proximal liegenden Schlaufe des Implantats fixiert sein, so dass das Fadenkreuz und damit die Membran in Richtung proximal gespannt werden, sobald das Implantat gestreckt wird. Selbstverständlich muss es sich nicht um lediglich zwei Fäden handeln, die ein Fadenkreuz ausbilden, ebenso denkbar sind nahezu beliebige andere Fadengeflechte, die eine Art Gerüststruktur ausbilden, um der Membran eine Struktur aufzuprägen.

Allgemein ist für die Erfindung wesentlich, dass die Trennzone ihre Funktion erfüllt, nämlich in das Aneurysma eingeführte Okklusionsmittel, etwa Okklusionsspiralen, zuverlässig zurückzuhalten oder den Blutstrom so abzulenken, dass weitere Okklusionsmittel unnötig sind. Die Trennzone verläuft orthogonal zur Längsachse des Implantats, wobei die die Trennzone ausbildenden Fasern, Fäden, Drähte etc. im Wesentlichen in einer Ebene liegen können.

Wird die Trennzone durch das Einziehen von Fasern, Fäden oder dünnen Drähten ausgebildet, ist die Anordnung von Ösen im Bereich der Trennzone zweckmäßig. Beispielsweise können die Maschen des Abschnitts (d) mit entsprechenden Ösen ausgestattet sein, in die Fäden kreuz- oder sternförmig eingeknotet werden. Die Ösen selbst können aus einem Fasermaterial gefertigt sein. Die Fäden/Fasern bestehen z. B. aus einem geeigneten Polymer wie einem Polyamid (Nylon) oder es handelt sich um metallische Fasern.

Die Trennzone kann aber auch durch aus einem Rohrmaterial geschnittene Bögen oder aus (Draht)schlaufen gebildet werden, wobei die Maschen des Abschnitts (d) nach außen umgeformt werden und die Bögen/Schlaufen der Trennzone in den Implantatkörper hinein umgebogen sind. Mindestens ein Bogen/eine Schlaufe ist erforderlich. Bei zwei bis vier Bögen/Schlaufen bilden diese ein stabiles Trennelement, das in ein Aneurysma eingebrachte Okklusionsmittel zuverlässig zurückhält.

Die Schlaufen können eine Wabenform aufweisen. Beim Kontrahieren des Implantats werden die Schlaufen typischerweise nach proximal gestreckt und legen sich an die weiteren Filamente des Implantats an, so dass sich das Implantat problemlos durch einen Katheter bewegen lässt.

Der distale Abschnitt (d) des erfindungsgemäßen Implantats ist insbesondere atraumatisch, weich und elastisch ausgebildet. Die Wandungen von Aneurysmen sind empfindlich und können bei Belastung reißen, was unter allen Umständen verhindert werden muss. Entsprechend muss insbesondere der distale Abschnitt (d) des erfindungsgemäßen Implantats atraumatisch gestaltet sein. Dies wird beispielsweise mit der Anordnung von Schlaufen erreicht, die sich dort, wo sie Kontakt mit der Aneurysmawandung bekommen, sanft an diese anschmiegen. Solche Schlaufen können, wie andere Bereiche des Implantats auch, durch Laserschneiden aus einem Rohr generiert, mittels angehefteter Drähte, die beispielsweise mit dem Abschnitt (c) laserverschweißt sind, oder aus einem einheitlichen Drahtgeflecht erzeugt werden. Diese Übergangszone deckt sich insbesondere mit der Trennzone, kann aber auch einen erweiterten Bereich des Abschnitts (c) darstellen, mit distal davon angeordneter Trennzone.

In jedem Fall ist es von Bedeutung, im distalen Abschnitt (d) alle Drahtenden atraumatisch auszubilden, um eine Perforation der Aneurysmawand zu verhindern.

Die Maschen im distalen Abschnitt (d) können als gerundete Bögen enden, insbesondere am distalen Ende aber auch Nasen (Vorsprünge) aufweisen, welche naturgemäß aber ebenfalls gerundet und atraumatisch sind. Diese Nasen haben den Effekt, dass sich das Implantat in der gestreckten Form im Katheter einfacher, d. h. mit geringerem Kraftaufwand, verschieben lässt.

Die erfindungsgemäßen Implantate können durchgängig die Form eines aus der Maschenstruktur gebildeten seitlich geschlossenen Rohrs haben, jedoch auch seitlich partiell oder durchgehend geschlitzt sein. Die Schlitzung kann achsparallel verlaufen oder schräg-/helixförmig. In solch einem Fall ist in den geschlitzten Bereichen die Maschenstruktur entsprechend der Gefäßform gerollt, etwa in der Form eines gerollten Segments eines Maschendrahtzauns. Bei der Implantation eines solchen geschlitzten Implantats erlaubt dies eine gute Anpassung an das Gefäßlumen, insbesondere des zuführenden Gefäßes, wobei eine geringfügige Unter- oder Überdeckung der seitlichen Ränder der Maschenstruktur in der Regel unproblematisch ist.

Möglich ist beispielsweise eine partielle Schlitzung, die am distalen Abschnitt (d) endet. Eine solche Schlitzung erlaubt eine gute Anpassung an den Gefäßverlauf, insbesondere im Bereich der Abschnitte (a) bis (c), und führt damit zu einer guten Fixierung des Implantats im Gefäß. Überraschend hat sich gezeigt, dass die Schlitzung keinen negativen Einfluss auf die Radialkraft ausüben muss.

Es ist möglich, zumindest einige Maschen des Implantats mit Unterbrechungen zu versehen, d. h. die Maschen sind zum Teil nicht vollständig geschlossen. Ein solches Open-Cell-Design weist eine höhere Flexibilität auf, was bei stark gewundenen Blutgefäßen von Vorteil sein kann. Darüber hinaus bewirkt das Fehlen von Stegen/Streben einen verbesserten Blutfluss im Bereich der Gefäßverzweigung. Allerdings wird der Vorteil der erhöhten Flexibilität damit erkauft, dass sich ein Implantat mit Open-Cell-Design schlechter in den Mikrokatheter zurückziehen lässt, wenn sich dies bei der Einführung als notwendig herausstellen sollte. Aus diesem Grund kann bei einer solchen Ausführungsform die proximale Anbindung an eine Einführhilfe über den Abschnitt (a) entfallen. Ein alternatives Einfuhrsystem kann beispielsweise so aussehen, dass das im Mikrokatheter radial komprimierte Implantat auf einem Draht zwischen zwei Nocken lagert und sich bei Entfernung des Mikrokatheters selbständig entfaltet und dadurch vom Einfuhrsystem löst.

Die erfindungsgemäßen Implantate weisen in der Regel röntgendichte Markerelemente auf, die die Visualisierung und Platzierung am Implantationsort erleichtern. Solche Markerelemente sind beispielsweise im Bereich des distalen Endes des Abschnitts (d) angeordnet, wobei sie bei zusammengeführten Drähten die Verbindungspunkte atraumatisch umformen können. Solche Markerelemente können auch in Form von Drahtwicklungen, als Manschetten und als geschlitzte Rohrabschnitte vorliegen, die auf das Implantat aufgecrimpt werden, beispielsweise im Übergangsbereich der Abschnitte (c) und (d) oder auf den Drahtschlaufen des Abschnitts (d). Für die Markerelemente kommen als Materialien insbesondere Platin und Platinlegierungen in Frage, beispielsweise eine Legierung aus Platin und Iridium, wie sie vielfach im Stand der Technik für Markerzwecke und als Material für Okklusionscoils eingesetzt wird. Idealerweise ist der distale Abschnitt (d) und sind insbesondere die Schlaufen vollständig oder teilweise röntgendicht, d. h. röntgensichtbar ausgebildet.

Möglich ist es auch, röntgensichtbare Substanzen in die Membranen einzubringen. Hierbei kann es sich um röntgensichtbare Partikel handeln, wie sie üblicherweise in der Röntgentechnik als Kontrastmittel eingesetzt werden. Solche röntgendichten Substanzen sind beispielsweise Schwermetallsalze wie Bariumsulfat oder lodverbindungen. Die Röntgensichtbarkeit der Membran ist bei der Einbringung und Lokalisierung des Implantats hilfreich und kann zusätzlich oder anstelle von Markerelementen Verwendung finden.

Ggf. kann ein Teil der Waben des Implantats von Stegen mit dünnerem Querschnitt gebildet werden, um die Flexibilität des Implantats zu erhöhen. Der Bereich liegt vorzugsweise im Abschnitt (b) und soll einem nicht regelmäßigen Verlauf des Blutgefäßes in der Fixierzone Rechnung tragen.

Die Implantate müssen keine rohrförmige Struktur haben, sondern können auch als gerollte "Matten" vorliegen, die sich gegen die Gefäßwand aufspannen. Auch eine partielle Schlitzung ist möglich.

Die Erfindung betrifft schließlich ein Implantat nach der vorstehenden Beschreibung, das an einen üblichen Führungsdraht angekoppelt ist. Diese Ankopplung kann beispielsweise durch Verbindungselemente erfolgen, die sich unter Einwirkung von elektrischem Strom elektrolytisch auflösen. Solche Verbindungselemente und Materialien sind insbesondere für die Ablösung von Okklusionsspiralen und Stents vielfach beschrieben. Auch eine mechanische Ablösung durch Kupplungselemente ist ohne Weiteres möglich, wobei die Kupplungselemente mit entsprechend angepassten Kupplungsteilen des Führungsdrahts zusammenwirken. Unter dem äußeren Zwang eines Katheters oder einer Hülle bleibt diese Verbindung intakt; nach Herausschieben des Implantats und der Kupplungsstelle aus dem Katheter oder der Umhüllung löst sich aber die Verbindung und setzt das Implantat mit den zum Implantat gehörigen Kupplungselementen frei.

Die Erfindung betrifft auch die Platzierung der erfindungsgemäßen Implantate im Blutgefäßsystem. Dies kann mit Hilfe eines üblichen Katheters oder Mikrokatheters erfolgen; diese Technik ist allgemein erprobt und wird vielfach angewandt. Sofern die Trennzone nicht bereits selbst für eine ausreichende Abdichtung des Aneurysmenhalses sorgt, werden nach Platzierung des Implantats Okklusionsmittel in das Aneurysma eingebracht. Hierzu wird das distale Ende eines Mikrokatheters durch die Trennzone hindurch in das Aneurysma geführt und die Okklusionsmittel, insbesondere Coils werden freigesetzt. Anschließend wird der Mikrokatheter zurückgezogen, die Okklusionsmittel werden durch das Implantat daran gehindert, aus dem Aneurysma zu entweichen. Neben herkömmlichen Okklusionsmitteln wie Coils können auch in anderer Weise geformte Körper zum Verschließen von Aneurysmen eingebracht werden, beispielsweise geflochtene oder auf andere Weise geformte Kugelkörper.

Die Erfindung wird durch die beiliegenden Abbildungen näher erläutert. Es zeigen:
- Figur 1: als Prinzipskizze ein Bifurkationsaneurysma;
- Figur 2: erfindungsgemäßes schematisch ein nicht Implantat, implantiert in den Bereich einer Gefäßverzweigung mit Bifurkationsaneurysma;
- Figur 3a: das Implantat mit seinen Sektionen als Prinzipdarstellung;
- Figur 3b: eine weitere Prinzipdarstellung des Implantats
- Figur 4: ein nicht erfindungsgemäßes Implantat, wie es gemäß Figur 2 eingesetzt werden kann;
- Figur 5: Varianten für den Abschnitt (d) eines Implantats;
- Figur 6: eine Ausführungsform eines Implantats, flächig ausgebreitet;
- Figur 7a: ein Implantat mit schlaufenförmigen distalen Abschnitten (d) in einer Ansicht von distal;
- Figur 7b: das Implantat aus Figur 7a in einer Seitenansicht;
- Figur 7c: ein nicht erfindungsgemäßes Implantat mit einer Membran in der Trennzone, die sich in Richtung proximal erstreckt;
- Figur 8: weitere Varianten eines nicht erfindungsgemäßenImplantats mit schlaufenförmigen distalen Abschnitten (d);
- Figur 9: ein Bifurkationsaneurysma mit aus dem Aneurysmabereich abzweigenden Seitengefäßen und einem implantierten Implantat;
- Figur 10: in flächig ausgebreiteter Form Varianten Implantate;
- Figur 11: eine weitere Variante mit einwärts- und auswärtsweisenden Bögen im Abschnitt (d);
- Figur 12: eine weitere Variante mit Gelenkverbindern im Abschnitt (c);
- Figur 13: eine weitere Variante eines Implantats mit erhöhter Flexibilität;
- Figur 14: alternative Ausführungsformen von nicht erfindungsgemäßen Implantaten;
- Figur 15: verschiedene Varianten von erfindungsgemäßen und nicht erfindungsgemäßen Implantaten in der Seitenansicht und in der Frontalansicht;
- Figur 16: eine weitere Variante des erfindungsgemäßen Implantats in der Seitenansicht und in der Frontalansicht;
- Figur 17: weitere Varianten des erfindungsgemäßen Implantats in flächig ausgebreiteter Form und
- Figur 18: eine weitere Variante eines nicht erfindungsgemäßen Implantats in der Frontalansicht.

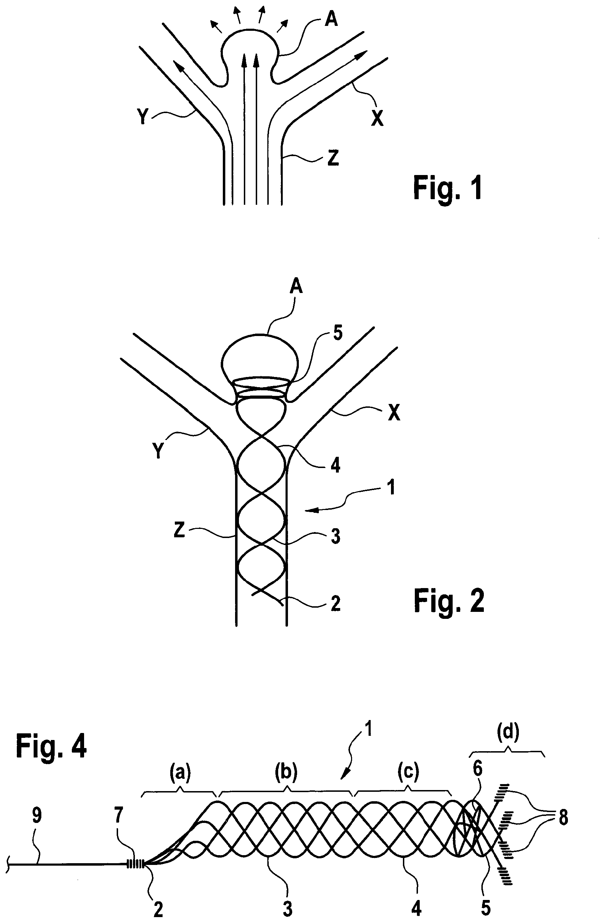

Figur 1 zeigt ein Bifurkationsaneurysma mit einem zuführenden Gefäß Z, zwei abführenden Gefäßen X und Y sowie dem in der Gabelung angeordneten Aneurysma A. Die langen Pfeile stellen den Blutstrom dar, der auf der Prallseite in das Aneurysma A einfließt und dort einen Druck nach außen ausübt, unter dem das Aneurysma sich erweitert (kleine Pfeile).

Figur 2 zeigt eine Gefäßkonstellation mit einem Aneurysma A, wie in Figur 1 beschrieben, mit einem darin angeordneten Implantat 1. Das Implantat hat ein proximales Ende 2, das das Kupplungselement aufweist

und vor der Ablösung in den Führungsdraht (nicht dargestellt) übergeht. Das Implantat 1 ist über seine Maschen 3 an der Gefäßwand des zuführenden Gefäßes Z verankert und weist im Bereich der Bifurkation Maschen 4 mit einer größeren Maschenweite auf. Im Hals des Aneurysmas ist ein distaler Bereich 5 dargestellt. Zwischen dem distalen Bereich 5 und dem Bereich mit den erweiterten Maschen 4 befindet sich eine Trennzone zur Rückhaltung von in das Aneurysma A nach Setzen des Implantats eingeführten Okklusionsmitteln.

Die erweiterten Maschen 4 im Bereich der Bifurkation erlauben es dem über das zuführende Gefäß Z einfließenden Blutstrom, in die Abzweigungen X und Y abzufließen, ohne groß behindert zu werden. Nach Setzen von hier nicht dargestellten Okklusionsmitteln im Aneurysma A ist der Blutstrom in das Aneurysma A so behindert, dass es dort zur Ausbildung eines Pfropfens und damit zur Stilllegung des Aneurysmas kommt. Alternativ kommt es zur Okklusion ohne Okklusionsmittel, sofern die Trennzone hinreichend dicht ist.

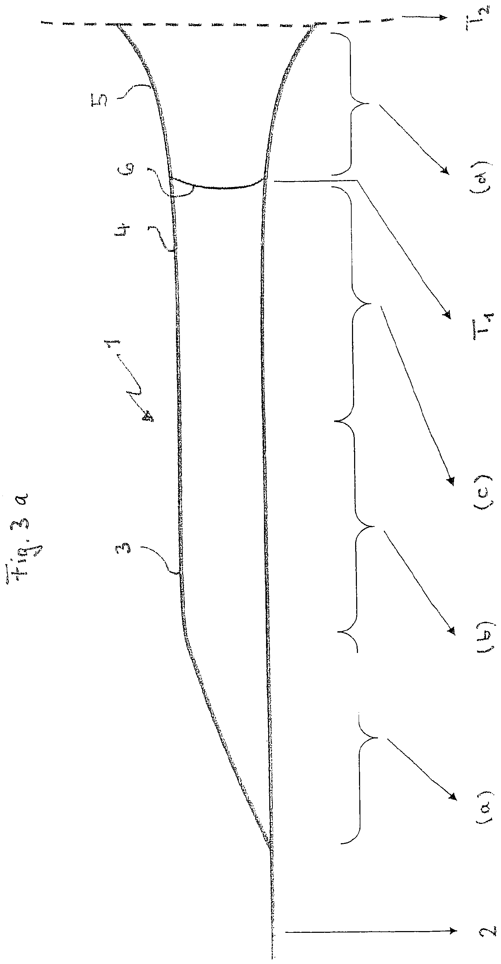

Figur 3a zeigt schematisch ein Implantat und seine Aufteilung in einzelne Abschnitte.

Das Implantat 1 hat einen proximalen Abschnitt (a), in dem sich das Implantat verjüngt und in einem Kupplungselement endet, hier als Draht dargestellt. Dieser Abschnitt entspricht dem Bereich 2 in Figur 2.

Daran schließt sich distal der Abschnitt (b) an, der der Fixierung des Implantats an der Gefäßwand des zuführenden Gefäßes Z dient. Dieser Bereich hat Maschen 3 mit einer relativ engen Maschenweite, die einen guten Kontakt zur Gefäßwandung herstellen.

Distal schließt sich dann der Abschnitt (c) an, der Maschen 4 mit einer relativ großen Weite aufweist. Dieser Bereich ist dazu bestimmt, einströmendes Blut in die Abzweigung X und Y, siehe Figur 1 und 2, abzugeben.

Das distale Ende des Implantats 1 ist der Abschnitt (d), in dem sich die Struktur 5 im gezeigten Fall trompetenförmig erweitert. Dieser Bereich kommt in dem Aneurysma A zu Liegen. Der Abschnitt (d) kann integraler Bestandteil des Implantats sein, d. h. zusammen mit den Abschnitten (a) bis (c) aus einem Rohr (Nitinol) geschnitten oder aus solchen Drähten geflochten sein. Es ist aber auch möglich, die Abschnitte (a) bis (c) aus einem Rohr zu schneiden und den Abschnitt (d) zu flechten und mit dem Abschnitt (c) zu verschweißen.

Zwischen den Abschnitten (c) und (d) ist die Trennzone T1 dargestellt, die ein oder mehrere Trennelemente 6 aufweist. Bei diesen Trennelementen kann es sich um eingespannte Fäden, Drähte oder Fasern, etwa aus Polyamid, handeln, aber auch um Teile einer geschnittenen Struktur, die nach innen umgeformt wurden. Diese Trennzone T1 mit den Trennelementen 6 dient dazu, in ein Aneurysma hinein platzierte Okklusionsmittel zurückzuhalten.

Je nach Art des Aneurysmas kann die Trennzone auch in den Abschnitt (d) hinein verschoben sein oder sich sogar am distalen Ende des Abschnitts (d) befinden. Eine solche Trennzone T2 ist insbesondere dann sinnvoll, wenn die Bifurkation so umgewandelt ist, dass die abzweigenden Gefäße X und Y nicht direkt vom zuführenden Gefäß Z abzweigen, sondern aus dem Aneurysma abzweigen. In diesem Fall muss die Trennzone unmittelbar oberhalb der Abzweigungen im sich erweiternden Abschnitt des Implantats angeordnet sein. Der Abschnitt (d) ist auf das distale Ende des Implantats 1 beschränkt und geht in die Trennzone T2 über.

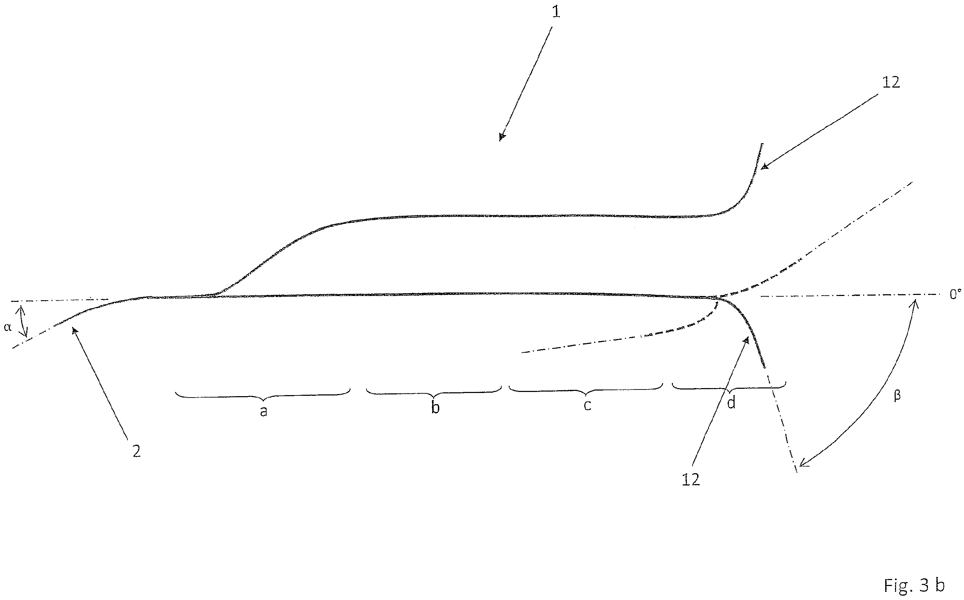

Figur 3b ist eine Darstellung des Implantats, die im Wesentlichen der aus Figur 3a entspricht. Darüber hinaus erkennt man, dass die hier nur schematisch wiedergegebenen Schlaufen 12 unterschiedliche Winkel zur Längsachse des Implantats 1 ausbilden können. Die Längsachse ist als gestrichelte Linie dargestellt. Der Winkel β kann sehr groß sein (> 90°, gestrichelte Darstellung), was insbesondere bei stark ausgewölbten Aneurysmen von Vorteil ist, wobei die Auswölbung zumindest partiell in Richtung proximal verläuft. In Extremfällen kann der Winkel β nahezu 180° betragen. Auf diese Weise wird eine gute Anschmiegung des distalen Abschnitts (d) an die Aneurysmenwand erreicht.

In anderen Fällen (ebenfalls gestrichelt dargestellt) kann es von Vorteil sein, wenn der Winkel β negativ ist, weil ein Teil der Aneurysmenwand nach innen gekrümmt ist. Wichtig ist, dass die Winkel für die einzelnen Schlaufen (12) sich unterscheiden können, was bei unregelmäßig geformten Aneurysmen von großem Vorteil ist.

Daneben ist in Figur 3b ebenfalls zu erkennen, dass das proximale Ende 2 des Abschnitts (a), an dem das Implantat in Kupplungsdrähten ausläuft, über die das Implantat mit einer Einführhilfe verbunden ist, einen Winkel a zur Längsachse des Implantats ausbildet. Dieser Winkel wird u. U. erst nach Implantation ausgebildet. Auf diese Weise wird eine verbesserte Expansion des Implantats und das Anlegen an die Blutgefäßwand gefördert. Ein unerwünschtes Hineinragen in das Blutgefäß wird vermieden.

Figur 4 zeigt ein nicht erfindungsgemäßes Implantat 1, wie es gemäß Figur 2 eingesetzt werden kann. Das Implantat 1 ist mit einem Führungsdraht 9 dargestellt und weist an seinem proximalen Ende 2 eine röntgendichte Markerspirale 7 auf. Das oder die Kupplungselemente, über die der Führungsdraht 9 mit dem Implantat 1 verbunden ist, sind nicht dargestellt, befinden sich aber im Bereich der Markerspirale 7.

Das dargestellte Implantat ist ein Geflecht aus einzelnen Drähten, die vorzugsweise aus Nitinol bestehen und denen die endgültige Implantatform aufgeprägt ist. Nitinol als Formgedächtnismaterial erlaubt es, das Implantat in komprimierter Form in einen Katheter zu führen, ohne dass die Formgebung verlorengeht. Nach der Freisetzung aus dem Katheter nimmt das Implantat die ihm aufgeprägte Form an, so dass es seinen Einsatzzweck erfüllen kann.

Das Implantat 1 ist in vier Abschnitte (a) bis (d) gegliedert, wobei der Abschnitt (a) den sich verjüngenden proximalen Abschnitt darstellt, der im proximalen Ende 2 zusammenläuft und in dem oder den Kupplungselementen endet. Der Abschnitt (b) ist ein Fixierabschnitt, der an der Gefäßwandung des zuführenden Gefäßes Z zu Liegen kommt. Der Abschnitt (c) ist durchlässig gestaltet mit Maschen 4, durch die der Blutstrom in die abzweigenden Gefäße X und Y austreten kann. Der Abschnitt (d) ist gegenüber dem Abschnitt (b) und hier auch gegenüber (c) erweitert und kommt im Aneurysma A zu Liegen. Die Enden der einzelnen Drähte sind durch Markerspiralen 8 aus einem röntgendichten Material, etwa Platin oder einer Platinlegierung, atraumatisch umgestaltet. Zwischen den Abschnitten (c) und (d) befindet sich ein Fasergeflecht 6, das beispielsweise aus Nylon gefertigt sein kann und das gleichzeitig die Trennzone T1 darstellt. Die Bezugsziffer 5 bezeichnet die sich nach außen erweiternden Maschen bzw. Filamente des Implantats 1 im distalen Bereich.

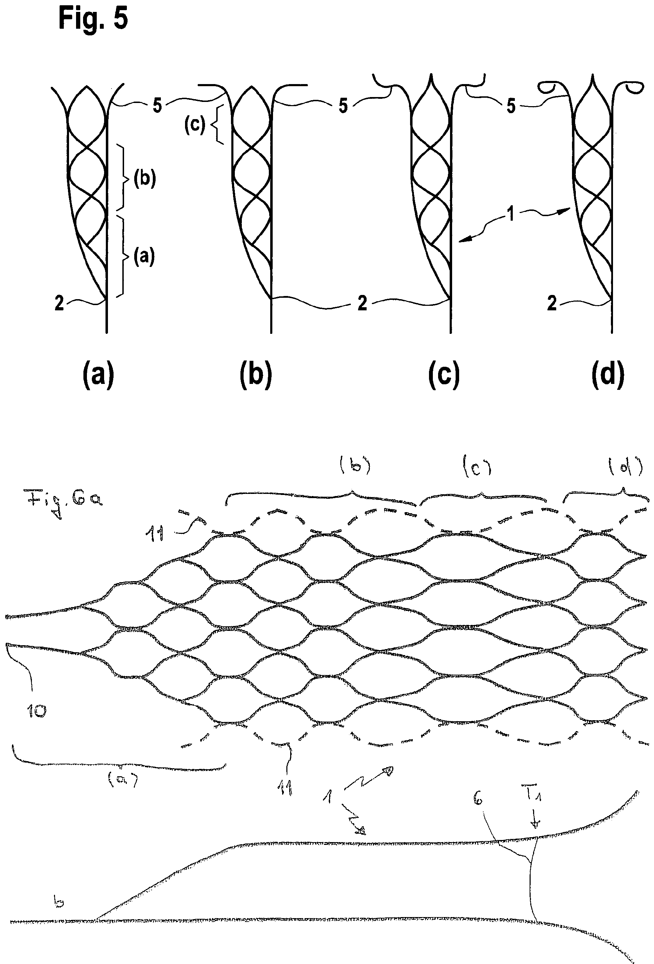

Figur 5 zeigt als Prinzipskizze vier Varianten der Ausgestaltung des distalen Bereiches 5 der Implantate 1. Figur 5a zeigt ein sich trompetenförmig aufweitendes distales Ende des Implantats, d. h. der Abschnitt (d) weitet sich kelchförmig auf. Gemäß Figur 5b ist das distale Ende 5 tellerförmig aufgeweitet, mit einem sehr eng begrenzten distalen Abschnitt (d). Figur 5c zeigt eine Kombination der Designelemente von Figur 5a und 5b.

Figur 5d zeigt schließlich einen distalen Bereich mit eingerollten distalen Enden der einzelnen Filamente eines Implantats 1. Zur Orientierung sind in Figur 5a bzw. 5b die Abschnitte (a), (b), (c) eingezeichnet.

Figur 6 zeigt in flächig ausgebreiteter Form eine Ausführungsform eines Implantats 1 mit den Abschnitten (a) bis (d). Das Implantat 1 ist als aus Nitinolrohr geschnittene Maschenstruktur zu verstehen, wobei in der Darstellung die gestrichelt gezeichneten Stege 11 den ausgezogenen Stegen auf der gegenüberliegenden Seite entsprechen. Deutlich zu erkennen sind die vergrößerten Waben im Bereich des Abschnitts (c) in der Darstellung von Figur 6a sowie die kelch- oder trompetenförmige Erweiterung in der Prinzipskizze gemäß Figur 6b. Dort ist auch die Trennzone T1 mit Trennelementen in Form einer eingezogenen Ebene von Nylonfäden 6 dargestellt.

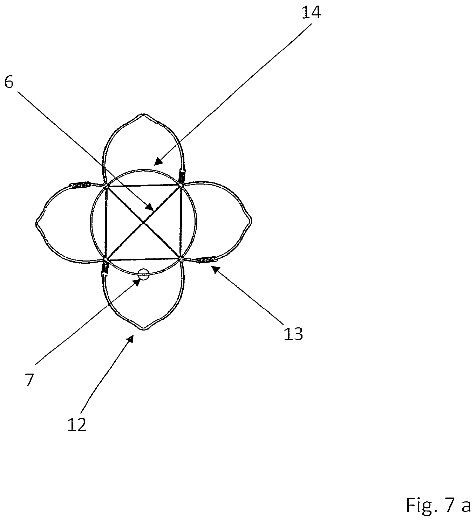

Figur 7a zeigt eine Ausführungsform des erfindungsgemäßen Implantats in der Ansicht von distal. Der distale Abschnitt (d) wird von Schlaufen 12 gebildet, die nach radial außen aufgedehnt sind. Die Trennzone 6 wird von einer Ebene aus Polymerfäden oder metallischen Fasern gebildet, die dafür sorgen, dass in das Aneurysma eingebrachte Okklusionsmittel dieses nicht wieder verlassen. Der Kreis 14 symbolisiert den Übergang in den zylindrischen Teil des Implantats. Darüber hinaus sind die Schlaufen mit röntgendichten Markerelementen 13 versehen.

Die Trennzone 6, d. h. der in der gewählten Darstellung quadratisch eingerahmte Bereich, und die Schlaufen 12 verfügen darüber hinaus über eine Membran, die wirkungsvoll den Bluteinstrom in das Aneurysma blockiert. Diese Membran kann an den Polymerfäden oder den metallischen Fasern sowie den Drähten der Schlaufen 12 fixiert sein, insbesondere können auch Polymerfäden oder metallische Fasern in die Membran eingebettet sein. Die Membran kann beispielsweise aus Polycarbonaturethan bestehen und mittels Elektrospinning gefertigt sein.

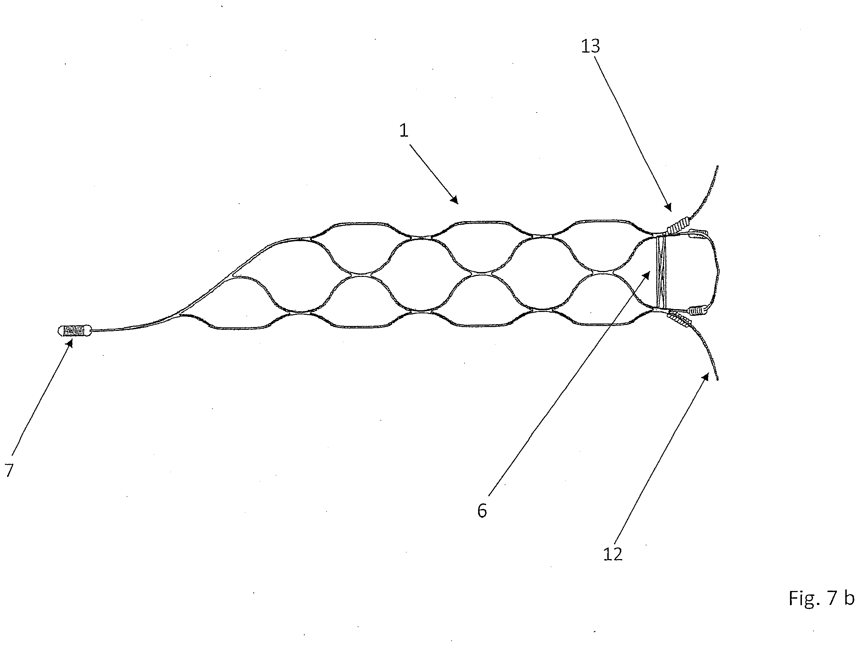

In Figur 7b ist das Implantat 1 aus Figur 7a von der Seite gezeigt. Man erkennt im distalen Bereich mehrere Schlaufen 12, die über Markerelemente 13 verfügen. Darüber hinaus wird der Eingang zum Aneurysma durch die Trennzone 6 blockiert, wobei es sich hier um sich kreuzende oder miteinander verwobene Polymerfäden oder metallische Fasern handeln kann, die den Austritt von in das Aneurysma eingebrachten Okklusionsmitteln verhindern. Ebenso sind die Schlaufen 12 und die Trennzone 6 mit einer Membran versehen, die das Aneurysma weitgehend vom Blutfluss abschneidet. In diesem Fall kann auf Okklusionsmittel im Aneurysma ggf. verzichtet werden. Schließlich verfügt das Implantat 1 am proximalen Ende über ein röntgendichtes Markerelement 7.

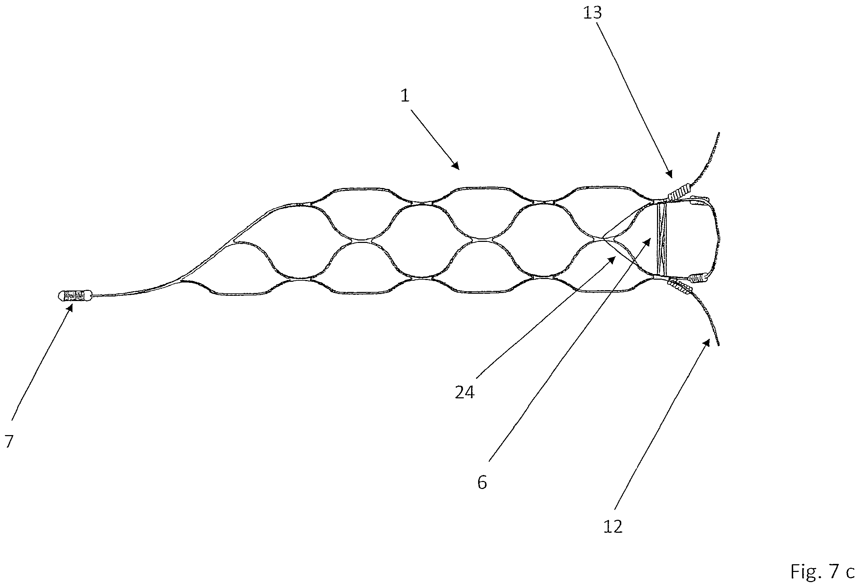

In Figur 7c schließlich ist eine weitere Ausführungsform mit einer Membran 24 in der Trennzone 6 dargestellt, wobei die Membran 24 eine Erstreckung in Richtung proximal aufweist. Die Membran 24 kann insbesondere kegel- oder pyramidenförmig sein, wobei der Scheitel des Kegels/der Pyramide proximal liegt. Um eine solche Membran aufzuspannen, ist eine Verstärkung der Membran 24 mit Fäden, Drähten oder Stegen des Implantats sinnvoll, die die Membran 24 in der gewünschten Position halten.

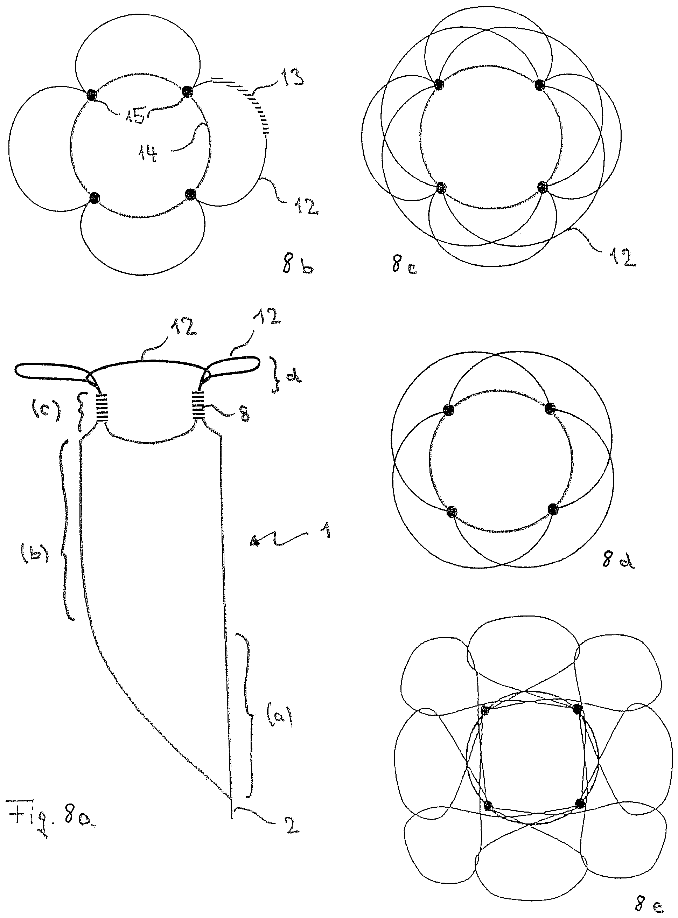

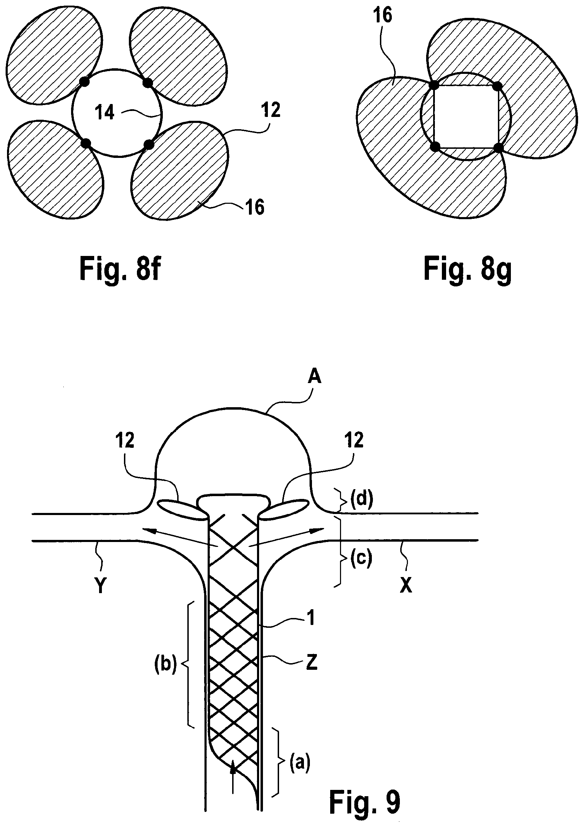

Figur 8 zeigt ein Implantat 1 mit einer eher tellerförmigen Ausgestaltung des Abschnitts (d), der im Wesentlichen aus Drahtschlaufen 12 besteht. Die Drahtschlaufen schließen sich an den zylindrischen Teil des Körpers des Implantats 1 an, wobei dieser zylindrische Teil von den Abschnitten (a) bis (c) gebildet wird. Im Übergangsbereich zu den angesetzten Schlaufen 12 befinden sich Markerelemente 8, die der sicheren Platzierung dienen. Im Bereich dieser Verbindung des zylindrischen Körpers des Implantats 1 und des Abschnitts (d) mit den Schlaufen 12 befindet sich der Abschnitt (c), der den Austritt des zufließenden Blutes in seitlich abgehende Gefäße erlaubt. Das Blut tritt somit zwischen den Stegen mit den Markerelementen 8 in die abgehenden Gefäße (X und Y, Fig. 2) ein.

Einzelne Varianten des distalen Abschnitts (d) sind in der Draufsicht in Figur 8b bis 8g dargestellt, wobei einzelne oder mehrere Schlaufen 12 mit Markerspiralen 13 versehen sein können. Die Markerspiralen 13 können die Schlaufen ganz oder teilweise umgeben. Die Schlaufen gehen im dargestellten Fall von vier Verbindungsstegen 15 aus, die auch die Markerelemente 8 tragen, wobei in den Darstellungen 9b bis 9g der innere Kreis 14 den Übergang in den zylindrischen Teil des Implantats darstellt. Etwaige vorhandene Verspannungen einer Trennzone T1 oder T2 sind nicht dargestellt.

Die Ausführungsformen gemäß Figur 8f und g zeigen mit einer dehnfähigen Membran 16 versehene Schlaufen 12, die in diesem Fall gleichzeitig eine Trennzone T2 ausbilden, wie in Figur 3 gezeigt. Die Figur 8f zeigt darüber hinaus, dass die Schlaufen 12 jeweils über lediglich einen Verbindungspunkt mit den weiteren Bereichen des Implantats verbunden sein können.

Es versteht sich, dass die Trennzonen T1 und T2 den zu okkludierenden Abschnitt des Aneurysmas A abteilen müssen. Je nach Typ des Aneurysmas liegt diese Trennzone dann im Eingangsbereich - bei proximal vom Eingangsbereich abgehenden Zweiggefäßen - oder aber innerhalb des Aneurysmas - dann, wenn zwei Gefäße aus dem Aneurysmaraum selbst abzweigen - wobei im letzteren Fall nur der von abzweigenden Gefäßen freie Teil des Aneurysmas okkludiert werden kann. Insbesondere bei tellerförmig ausgebildeten distalen Abschnitten (d) der Implantate kann sich, insbesondere bei größerer Anzahl der Drahtschlaufen, eine zusätzliche Verspannung oder Anordnung aus dem Rohr geschnittener Trennelemente erübrigen.

Die in Figur 8 dargestellten schlaufenförmigen distalen Abschnitte (d) können zum einen, wie auch der übrige Implantatkörper, aus einem Rohr mit dafür geeignetem Durchmesser geschnitten werden. Es ist aber auch möglich, die Abschnitte (a) bis (c) des Implantatkörpers aus einem Rohr in üblicher Art und Weise zu schneiden und den Abschnitt (d) aus Drahtfilamenten anzuheften, beispielsweise durch Laserverschweißen.

Figur 9 zeigt den Spezialfall eines Aneurysmas A, bei dem die abzweigenden Gefäße X und Y aus dem Aneurysma abgehen. Für diesen Fall sind die in Figur 8 beschriebenen Implantate 1 besonders geeignet, bei denen die Schlaufen 12 gleichzeitig die Trennzone T2 bilden, die im Aneurysma selbst distal von den abzweigenden Gefäßen angeordnet sind. Der zylindrische Körper des Implantats 1 mit den Abschnitten (a) und (b) befindet sich im zuführenden Gefäß Z, der Abschnitt (c), der den Durchtritt des Bluts in die Abzweigungen X und Y erlaubt, liegt im Bereich dieser Abzweigung und mittelbar distal dieses Abschnitts (c) befindet sich der Abschnitt (d) mit den Schlaufen 12. Die Schlaufen sind mit einer Membran bespannt, wobei diese Membran aus einem dehnfähigen Material besteht, beispielsweise Teflon, oder einem Faservlies. Ein solches Faservlies aus Polycarbonaturethan ist aus der DE 28 06 030 bekannt und zeichnet sich durch eine hohe Elastizität aus, die für die Applikation des Implantats durch einen Katheter günstig ist. Die Membran kann geschlitzt, gefaltet oder porös gestaltet sein, beispielsweise auch um Material einzusparen und den Transport durch einen Katheter zu erleichtern.

Eine derartige Membran kann auch als Trennelement für die Trennzone verwandt werden, wie sie zwischen den Abschnitten (c) und (d) angeordnet ist.

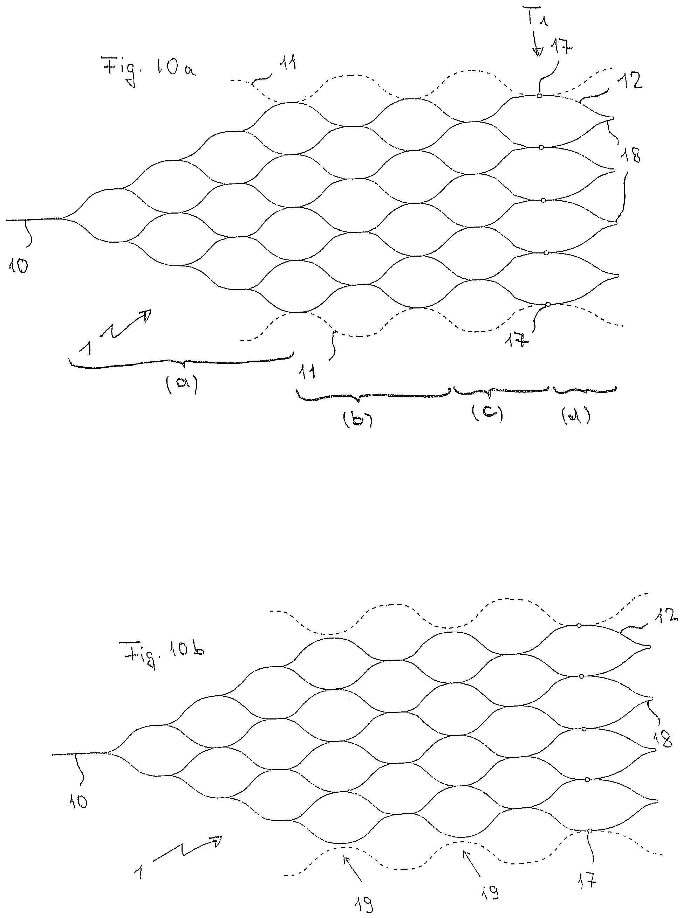

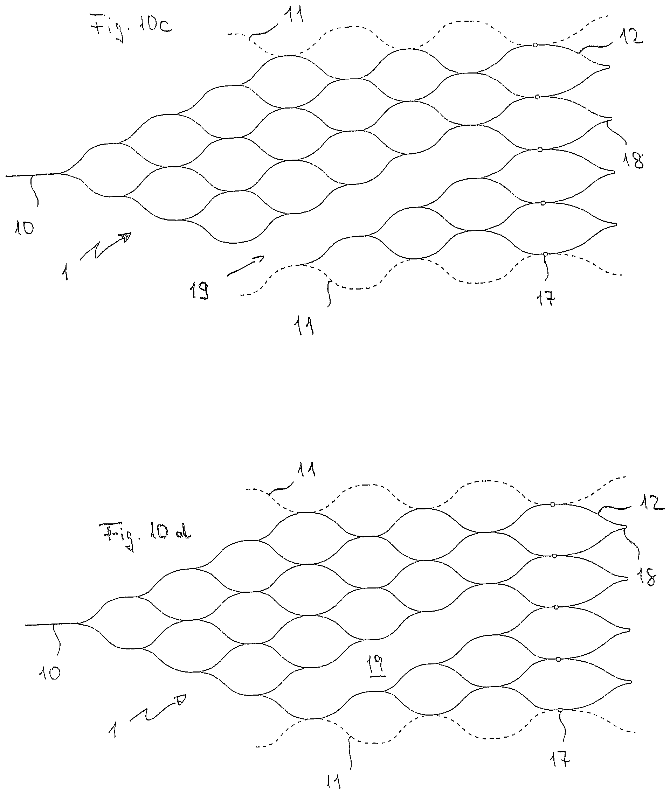

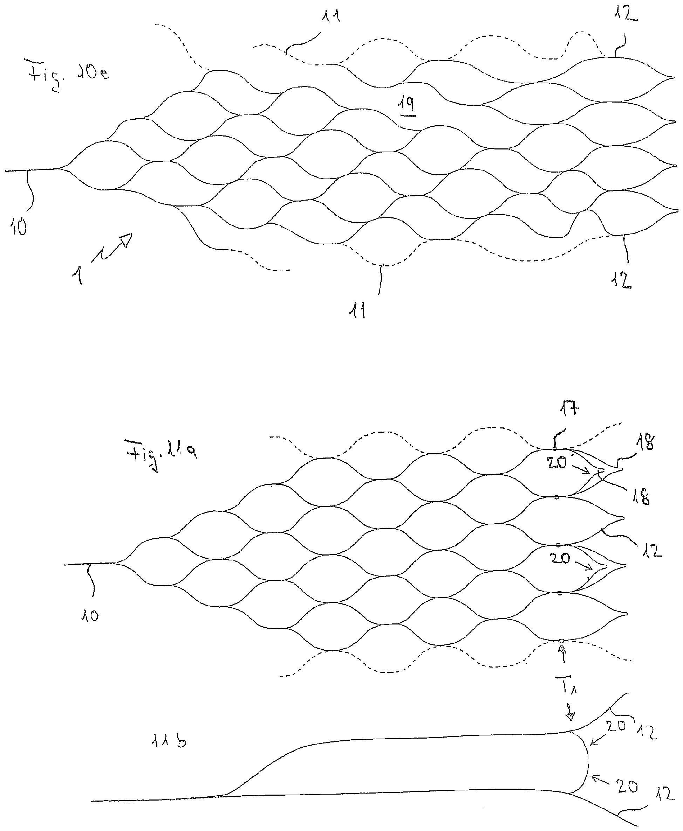

Figur 10 zeigt in flächig ausgebreiteter Form mehrere bevorzugte Ausführungsformen eines erfindungsgemäßen Implantats 1, bei der die Wabenstruktur durch im wesentlichen gleichmäßig große Waben ausgebildet wird; lediglich die distalen Schlaufen haben eine größere Wabenfläche.

Wie in Figur 6a entsprechen die gestrichelt gezeichneten Stege 11 den ausgezogenen Stegen auf der gegenüberliegenden Seite. Das Implantat 1 entspricht damit einem Rohr mit einer Gitter- oder Wabenstruktur.

An das proximal angeordnete Kupplungselement 10 schließt sich der proximale Abschnitt (a), daran der Fixierabschnitt (b) an. Der distale Abschnitt (d) beginnt im Bereich der Ösen 17, die zur Aufnahme und Fixierung von Draht- oder Nylonelementen dienen, mit denen in das Implantat eine Trennzone eingezogen wird. Die distalen Schlaufen im sich nach außen erweiternden Abschnitt (d) weisen distal Nasen auf, die sich als vorteilhaft bei der Einführung des Implantats durch einen Katheter am Einsatzort erwiesen haben.

Figur 10b entspricht in allen wesentlichen Punkten der Darstellung von Figur 10a, ausgenommen einer partiellen Schlitzung im Bereich der Pfeile 19, wo die Rohrstruktur des Implantats 1 nicht geschlossen ist. Die Schlitzung verläuft achsparallel und endet vor dem distalen Abschnitt (d), dort wo sich der durchlässige Abschnitt (c) befindet.

Figur 10c zeigt eine Variante mit einem nicht achsparallel verlaufenden Schlitz 19, der sich um die Längsachse windet, ebenfalls jedoch vor dem distalen Abschnitt (d) endet.

Derartige Schlitzungen haben sich als ausgesprochen vorteilhaft für die Flexibilität im Bereich der Fixierzone (b) erwiesen. Die Radialkraft des Implantats 1 wird hierdurch nicht wesentlich beeinträchtigt, jedoch die Anpassung an den Gefäßverlauf und das Gefäßlumen verbessert.

Figur 10d zeigt ebenfalls ein Implantat mit Schlitzung, wobei jedoch die Schlitzung sich nicht bis zu den Implantaträndern erstreckt.

Figur 10e zeigt eine weitere Variante mit einem Schlitz 19, der sich ebenfalls um die Längsachse windet, wobei allerdings Wabenformen nebeneinander existieren. Die Wabenform hat einen Einfluss auf die Flexibilität und kann je nach Anforderung gewählt werden.

Die Schlaufen bzw. Waben des distalen Abschnitts (d) sind in Figur 10 jeweils mit der Bezugsziffer 12 bezeichnet.

Figur 11 zeigt eine weitere Variante eines Implantats 1 mit einem einzelnen Kupplungselement 10 und einer im Wesentlichen regelmäßigen Wabenstruktur, bei dem als Trennelemente zusätzliche Schlaufen 20 vorgesehen sind. Die zusätzlichen Schlaufen 20 sind beim implantierten Produkt nach innen gerichtet und bilden die Trennzone T1 aus. Auch diese Schlaufen 20 weisen Nasen 18 auf, die den Transport durch einen Katheter erleichtern.

Figur 11b zeigt schematisch das Implantat von Figur 11a mit den einwärts weisenden Schlaufen 20 und der Trennzone T1.

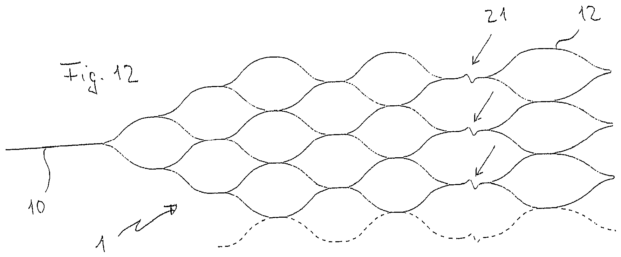

Figur 12 zeigt eine weitere Variante eines besonders flexiblen Implantats 1 mit Gelenkverbindern 21 in Form einer Zickzackführung der entsprechenden Stege zur Verbesserung der Anpassung des Implantats 1 an gekrümmt verlaufende Gefäße im Bereich der Bifurkation.

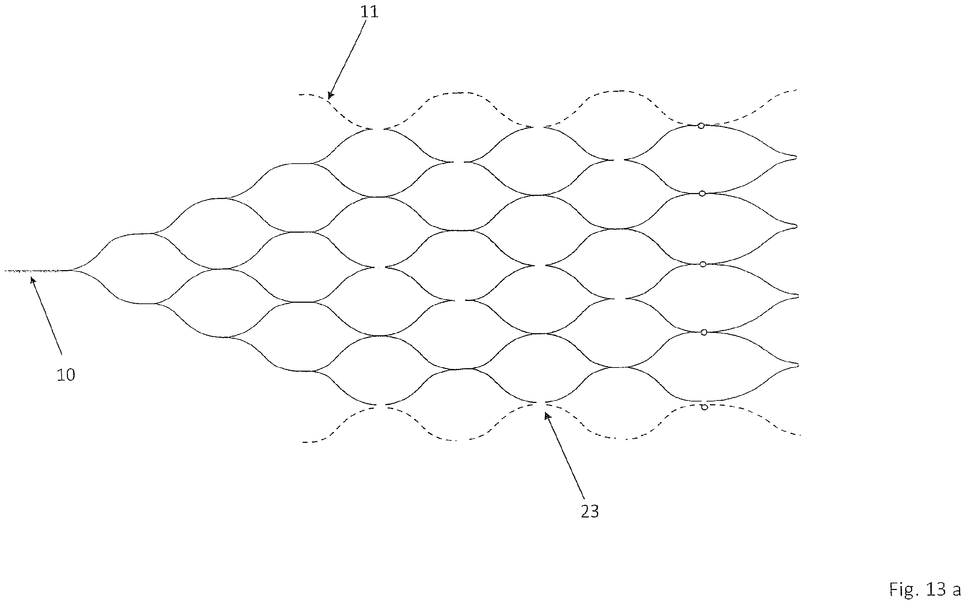

Figur 13a zeigt eine weitere Variante, bei der wie zuvor die gestrichelt gezeichneten Stege 11 den ausgezogenen Stegen auf der gegenüberliegenden Seite entsprechen. Die Ausführungsform zeichnet sich dadurch aus, dass die Wabenstruktur teilweise durchbrochen ist, d. h. einige Waben weisen Unterbrechungen 23 bzw. Lücken auf. Es ist möglich, sämtliche Waben mit Unterbrechungen 23 zu versehen, im vorliegenden Fall weisen jedoch nur einige Waben eine Unterbrechung 23 auf. Darüber hinaus ist es möglich, zwischen den Abschnitten zu variieren. In der hier gewählten Darstellung weisen lediglich die Waben im Abschnitt (c), nicht jedoch die Waben im Abschnitt (b) Unterbrechungen 23 auf. Am proximalen Ende ist das Implantat 1 über ein Kupplungselement 10 an die Einführhilfe angebunden.

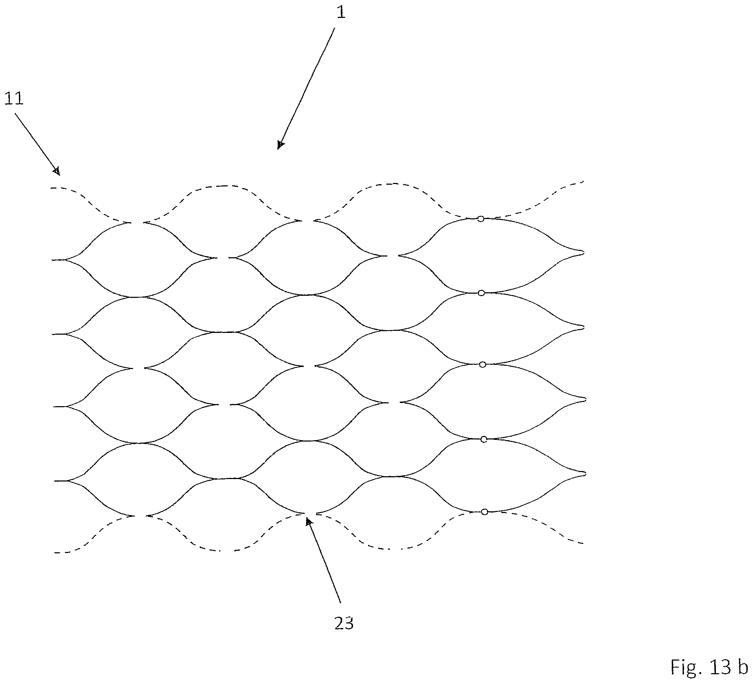

Da ein Zurückziehen eines mit Unterbrechungen versehenen Implantats 1 in den Katheter problematisch ist, kann auf die Zusammenführung des Implantats in Kupplungslemente am proximalen Ende ggf. auch verzichtet werden. Eine solche Ausführungsform ist in Fig. 13b dargestellt. Das Implantat 1 kann sich bei Ausschieben aus dem Mikrokatheter selbständig entfalten.

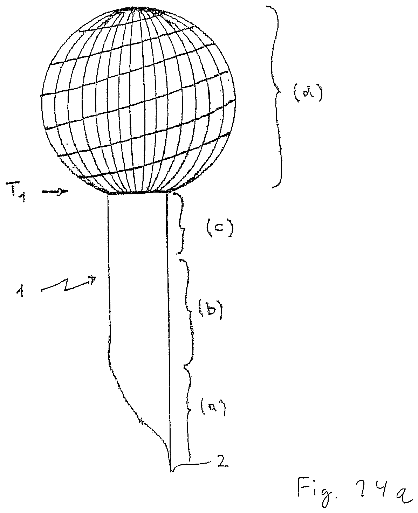

Figur 14a zeigt eine Ausführungsform eines nicht erfindungsgemäßen Implantats 1, das sich durch die besondere Ausgestaltung des distalen Abschnitts (d) auszeichnet, der als aus einzelnen Drähten oder Filamenten gebildete Kugel gestaltet ist. Vom proximalen Ende 2 des Implantats 1 an sind die Abschnitte (a) bis (c) ausgeführt, wie zuvor beschrieben. Zwischen den Abschnitten (c) und (d) befindet sich die Trennzone T1 mit Sperrelementen, wie sie zuvor beschrieben sind. Statt einer geschlossenen Kugel kann auch ein nach distal offener Korb für den Abschnitt (d) verwendet werden. Für die Kugel oder den Korb ist eine geflochtene Struktur bevorzugt.

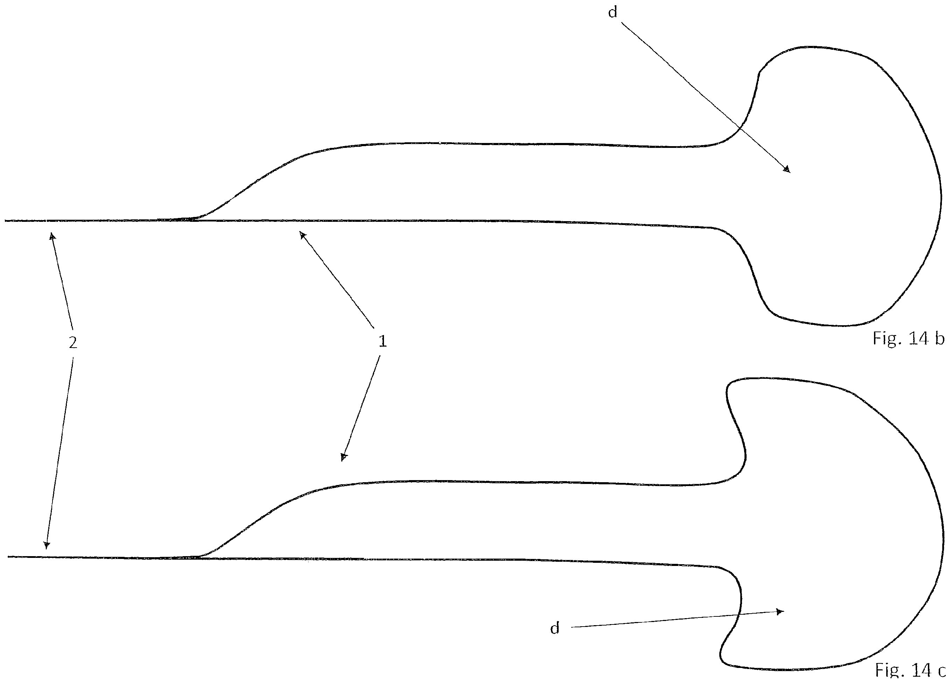

In den Figuren 14b und c sind weitere alternative Ausführungsformen für den distalen Abschnitt (d) dargestellt, welche als kugel- oder pilzförmig bezeichnet werden können, wobei die Form nicht regelmäßig sein muss. Insbesondere weicht die Form gemäß Fig. 14b von einem perfekten Kreis ab, ist aber in der Lage, sich gut an die Gefäßinnenwand anzuschmiegen. Die in Fig 14c gewählte Ausführungsform eignet sich besonders für ungewöhnlich geformte Aneurysmen, bei denen die Seitenwände stark ausgewölbt sind und partiell in Richtung proximal verlaufen.

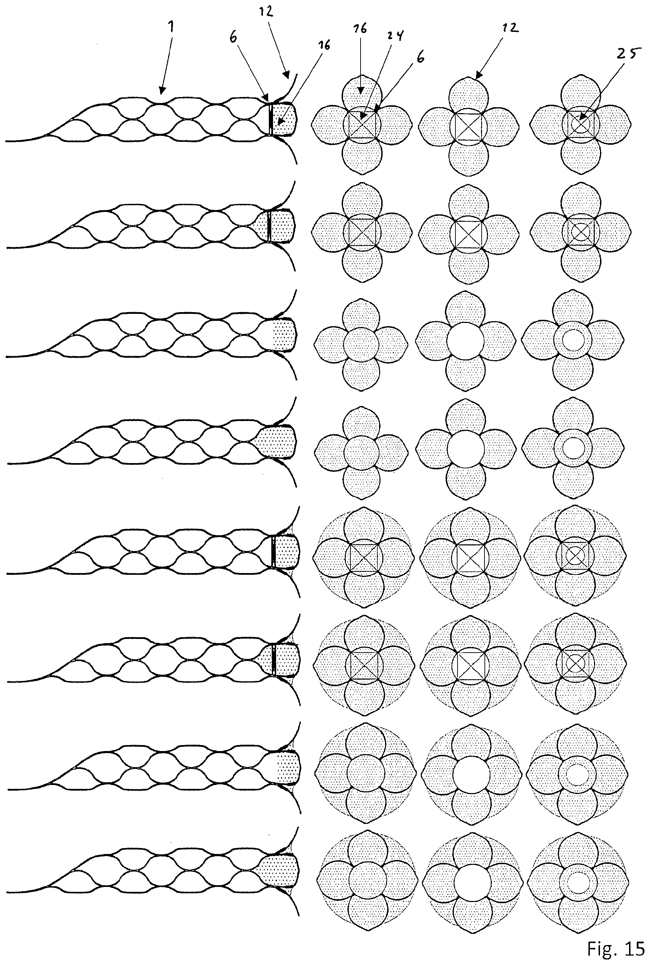

Figur 15 zeigt eine ganze Reihe verschiedener Ausführungsformen von erfindungsgemäßen und nicht erfindungsgemäßen Implantaten 1 in der Seitenansicht und einer Ansicht von distal, bei denen Membranen 16, 24 vorgesehen sind. Die Membranen 16 füllen das Innere der Drahtschlaufen 12 aus, die Membran 24 bildet (teilweise) die Trennzone 6, wobei die einzelnen Membranen 16, 24 ineinander übergehen können oder es sich um eine einzelne, Drahtschlaufen 12, Trennzone 6 und ggf. weitere Bereiche überspannende Membran handeln kann. Der mit einer Membran 16, 24 versehene Bereich ist gepunktet dargestellt. Wie man erkennt, sind auch Bereiche außerhalb der Drahtschlaufen 12 mit der Membran überspannt.

Man erkennt, dass es erfindungsgemäße Ausführungsformen gibt, bei denen lediglich die Drahtschlaufen 12 eine Membran aufweisen, während die Trennzone 6 von sich überkreuzenden Fäden/Drähten gebildet wird. Zusätzlich kann aber auch die Trennzone 6 eine Membran 24 aufweisen, wobei diese Membran durch eine Fadenstruktur gestützt werden kann, was aber nicht unbedingt erforderlich ist. Die Innenfläche der Drahtschlaufen 12 kann ganz oder teilweise mit der Membran 16 ausgefüllt sein.

Insbesondere sind auch Ausführungsformen möglich, bei denen die die Trennzone 6 ausbildende Membran 24 über Öffnungen 25 verfügt, so dass die Membran 24 zwar dicht genug ist, um das Austreten von Okklusionsmitteln zu verhindern, andererseits aber ein Mikrokatheter durch die Öffnung 25 in das Aneurysma eingeführt werden kann, um dort die Okklusionsmittel zu platzieren. Sofern im Bereich der Trennzone 6 des Weiteren sich überkreuzende Fäden/Drähte vorgesehen sind, muss ein ausreichender Zwischenraum verbleiben, der das Hindurchführen eines Katheters erlaubt.

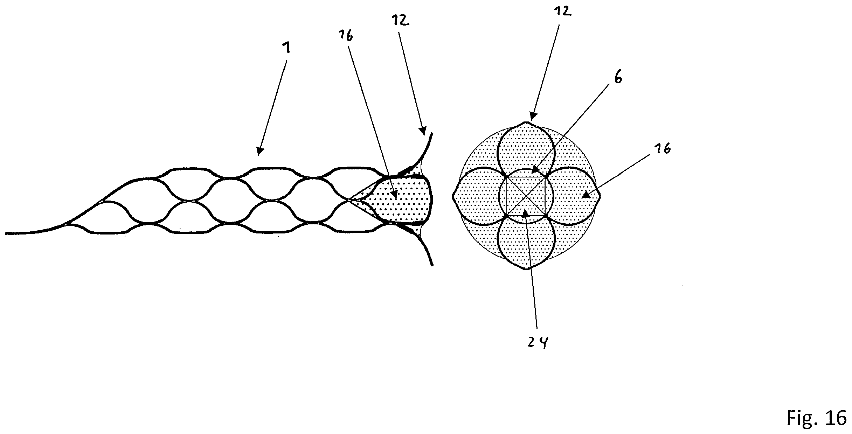

In Figur 16 ist eine ähnliche Darstellung einer alternativen Ausführungsform des erfindungsgemäßen Implantats 1 gezeigt, bei der die Membran 24, die die Trennzone 6 ausbildet, pyramidenförmig in Richtung proximal ausgerichtet ist. Auf diese Weise kann bewirkt werden, dass der Blutstrom seitwärts, d. h. vom zentral liegenden Aneurysma weggeleitet wird. Die Membran 24 wird von einer Fadenstruktur gehalten, die eine Fixierung proximal der Trennzone 6 aufweist, um auf diese Weise die Pyramidenform herbeizuführen. Sobald das Implantat 1 in einen Katheter eingezogen wird, wird die Membran 24 ebenfalls weiter nach proximal gezogen und klappt zusammen, was mit einer geringen Querschnittsbeladung einhergeht. Trotz einer ausreichend dichten Trennzone 6 im expandierten Zustand wird somit ein Implantat 1 geschaffen, das sich problemlos durch einen geeigneten Katheter manövrieren lässt.

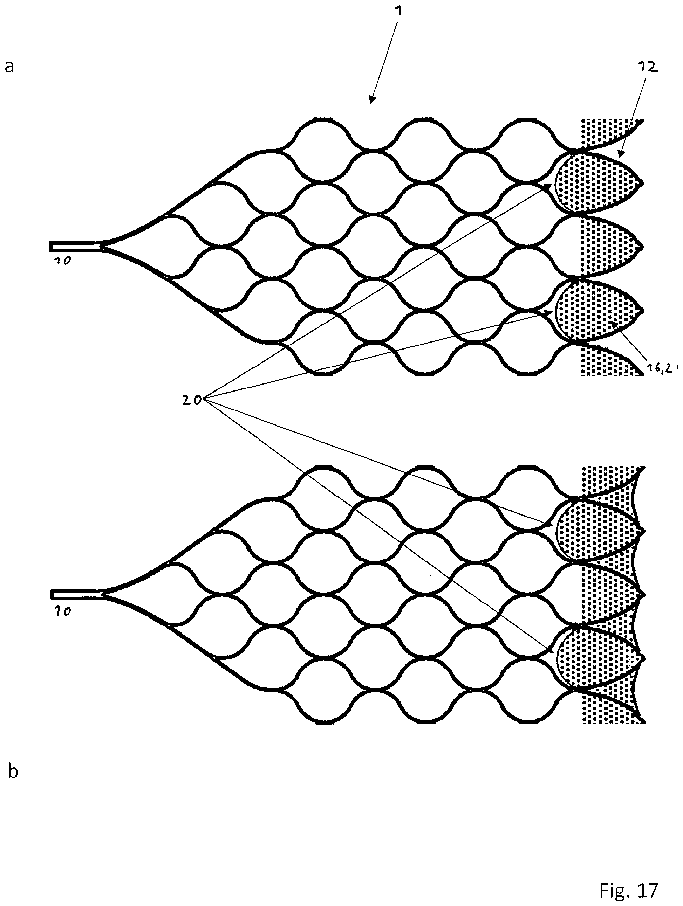

In Figur 17 schließlich wird eine Ausführungsform dargestellt, bei der, ähnlich Figur 11, die Trennzone 6 durch in den Innenraum weisende Drahtschlaufen 20 gebildet wird. Als Darstellung wurde eine auseinandergefaltete Implantatstruktur in der Seitenansicht gewählt. In der Darstellung sind zwei nach innen ragende Drahtschlaufen 20 dargestellt, grundsätzlich können aber auch mehr Drahtschlaufen 20 vorgesehen sein. Die Drahtschlaufen 20 sind, ebenso wie die nach distal außen weisenden Drahtschlaufen 12 mit einer Membran 16, 24 versehen, die im Bereich der Trennzone 6 deren Dichtigkeit weiter erhöht. In Fig. 17b sind darüber hinaus auch Zwischenräume zwischen den Drahtschlaufen 12 mit einer Membran 16 versehen.

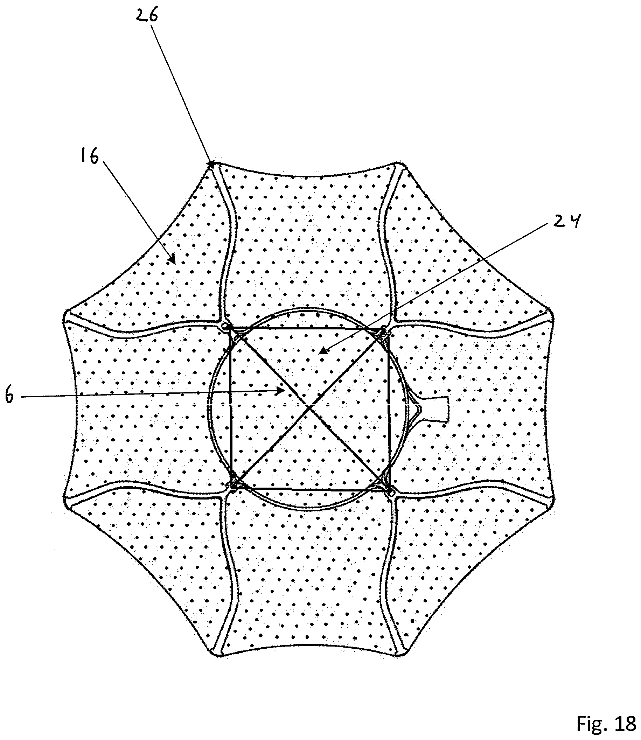

In Figur 18 ist eine Frontalansicht einer nicht erfindungsgemäßen Ausführungsform dargestellt. Die Darstellung ähnelt der in Figur 15 gewählten, anstelle von Schlaufen sind hier allerdings radial nach außen weisende Streben 26 vorgesehen, die Abschnitt (d) ausbilden. Die Streben 26 laufen konzentrisch zusammen, wobei jeweils zwei Streben 26 eine Einheit ausbilden und einen gemeinsamen Ursprung am distalen Ende des Abschnitts (c) aufweisen. Die Streben 26 dienen auch dazu, eine gepunktet dargestellte Membran 16, 24 aufzuspannen, die sich sowohl über den Innenbereich der Trennzone als auch über die Zwischenräume zwischen den Streben 26 erstreckt. Eine zusätzliche Stabilisierung kann durch eine Fadenstruktur 6 in der Trennzone erfolgen, ist aber nicht unbedingt erforderlich. Allerdings erleichtert die Fadenstruktur 6 ein Durchstechen einzelner Segmente der inneren Membran 24, während gleichzeitig andere Bereiche der Membran 24 unbeschädigt bleiben, so dass Okklusionsmittel in das Aneurysma eingeführt werden können.

## Patentansprüche

1. Implantat zum Einsatz bei der Okkludierung von Aneurysmen im Bereich von Gefäßverzweigungen, insbesondere Bifurkationsaneurysmen (A), mit einer Maschenstruktur (3, 4), welches - von proximal nach distal - die Abschnitte (b) bis (d) aufweist:
(b) einen Fixierabschnitt, mit dem das Implantat an einer Gefäßwand abstützbar ist,
(c) einen durchlässigen Abschnitt für den Bereich der Gefäßverzweigung und
(d) einen distalen Abschnitt, in dem das Implantat gegenüber dem Abschnitt (b) radial erweitert ist und der zur Platzierung im Aneurysma (A) bestimmt ist,
wobei im Bereich der Abschnitte (c) oder (d) eine Trennzone (T1, T2) angeordnet ist, die so konfiguriert ist, dass sie den Hals des Aneurysmas zumindest teilweise verschließt,
der distale Abschnitt (d) eine Mehrzahl von mit dem Abschnitt (c) verbundenen Schlaufen (12) aufweist und die Schlaufen (12) zur Längsachse des Implantats (1) einen Winkel zwischen -45° und +175° ausbilden, wobei ein positiver Winkel für nach radial außen und ein negativer Winkel für nach radial innen weisende Schlaufen (12) steht,
**dadurch gekennzeichnet,**
**dass** die Trennzone eine oder mehrere Membranen (16, 24) aufweist, die das Innere der Schlaufen (12) ausfüllen und Zwischenräume zwischen den Schlaufen (12) überspannen.

2. Implantat nach Anspruch 1, **dadurch gekennzeichnet, dass** die oder die Membranen (16, 24) im Bereich der Trennzone (T1, T2) orthogonal zur Längsachse des Implantats (1) aufgespannt sind.

3. Implantat nach einem der Ansprüche 1 bis 2, **dadurch gekennzeichnet, dass** sich die Membranen (16, 24) in proximaler Richtung erstrecken und vorzugsweise eine Kegel- oder Pyramidenform ausbilden.

4. 6. Implantat nach einem der Ansprüche 1 bis 3, **dadurch gekennzeichnet, dass** die Schlaufen (12) zur Längsachse des Implantats (1) einen Winkel zwischen +45° und +90° ausbilden.

5. Implantat nach einem der Ansprüche 1 bis 4, **dadurch gekennzeichnet, dass** die Schlaufen (12) im distalen Abschnitt (d) Ösen (17) aufweisen.

6. 8. Implantat nach einem der Ansprüche 1 bis 5, **dadurch gekennzeichnet, dass** die Schlaufen (12) im distalen Abschnitt (d) Nasen (18) am distalen Ende aufweisen.

7. Implantat nach einem der Ansprüche 1 bis 6, **dadurch gekennzeichnet, dass** die Schlaufen (12) über einzelne, insbesondere jeweils ein oder zwei Verbindungspunkte am Abschnitt (c) festgelegt sind.

8. Implantat nach einem der Ansprüche 1 bis 7, **dadurch gekennzeichnet, dass** das Implantat (1) proximal des Abschnitts (b) einen Abschnitt (a) aufweist, wobei der Abschnitt (a) ein sich verjüngender proximaler Abschnitt ist, in dem die Maschenstruktur (3, 4) zu einem oder mehreren Kupplungselementen (10), vorzugsweise Kupplungsdrähten, zusammengeführt ist.

9. Implantat nach Anspruch 8, **dadurch gekennzeichnet, dass** die Kupplungselemente (10) exzentrisch auf dem Umfang des Implantats (1) in seiner expandierten Form zusammengeführt werden.

10. Implantat nach Anspruch 9, **dadurch gekennzeichnet, dass** die Kupplungselemente (10), vorzugsweise Kupplungsdrähte, zur Längsachse des Implantats (1) einen Winkel zwischen 0° und +60°, vorzugsweise +10 bis +30°, ausbilden, wobei ein positiver Winkel für nach außen weisende Kupplungselemente (10) steht.

11. Implantat nach einem der Ansprüche 1 bis 10, **dadurch gekennzeichnet, dass** die Trennzone (T1, T2) Trennelemente aus orthogonal zur Längsachse des Implantats (1), vorzugsweise im Wesentlichen in einer Ebene verlaufenden Filamenten (6) aufweist.

## Claims

1. Implant for use for the occlusion of aneurysms in the region of vessel branches, in particular bifurcation aneurysms (A), with a mesh structure (3, 4) which - from proximally to distally - comprises the following portions (b) to (d):
(b) a fixing portion with which the implant can be supported on a vessel wall,
(c) a permeable portion for the region of the vessel branch, and
(d) a distal portion in which the implant is radially widened with respect to the portion (b) and which is intended to be placed in the aneurysm (A),
wherein a separating zone (T1, T2) which is configured such that it at least partly closes off the neck of the aneurysm is arranged in the region of the portions (c) or (d),
the distal portion (d) comprises a plurality of loops (12) connected to the portion (c) and the loops (12) form an angle of between -45° and +175° relative to the longitudinal axis of the implant (1), wherein a positive angle stands for loops (12) pointing radially outwards and a negative angle for ones pointing radially inwards,
**characterised in that**
the separating zone comprises one or more membranes (16, 24) which fill the interior of the loops (12) and bridge gaps between the loops (12).

2. Implant according to claim 1, **characterised in that** the membrane(s) (16, 24) in the region of the separating zone (T1, T2) are spanned orthogonally to the longitudinal axis of the implant (1).

3. Implant according to one of claims 1 to 2, **characterised in that** the membranes (16, 24) extend in the proximal direction and preferably form a conical or pyramid shape.

4. Implant according to one of claims 1 to 3, **characterised in that** the loops (12) form an angle of between +45° and +90° relative to the longitudinal axis of the implant (1).

5. Implant according to one of claims 1 to 4, **characterised in that** the loops (12) have eyelets (17) in the distal portion (d).

6. Implant according to one of claims 1 to 5, **characterised in that** the loops (12) in the distal portion (d) have noses (18) at the distal end.

7. Implant according to one of claims 1 to 6, **characterised in that** the loops (12) are secured to the portion (c) by way of individual, in particular in each case one or two, connecting points.

8. Implant according to one of claims 1 to 7, **characterised in that** the implant (1) proximally to the portion (b) has a portion (a), wherein the portion (a) is a tapering proximal portion in which the mesh structure (3, 4) is brought together to form one or more coupling elements (10), preferably coupling wires.

9. Implant according to claim 8, **characterised in that** the coupling elements (10) are brought together eccentrically on the periphery of the implant (1) in its expanded form.

10. Implant according to claim 9, **characterised in that** the coupling elements (10), preferably coupling wires, form an angle of between 0° and +60°, preferably +10 to +30°, relative to the longitudinal axis of the implant (1), wherein a positive angle stands for outward-pointing coupling elements (10).

11. Implant according to one of claims 1 to 10, **characterised in that** the separating zone (T1, T2) comprises separating elements, preferably of filaments (6) running orthogonally to the longitudinal axis of the implant (1), preferably substantially in one plane.

## Revendications

1. Implant destiné à être utilisé lors de l'occlusion d'anévrismes dans la région de ramifications de vaisseaux, en particulier d'anévrismes de bifurcation (A), avec une structure à mailles (3, 4), lequel présente - du côté proximal vers le côté distal, les parties (b) à (d) :
(b) une partie de blocage, avec laquelle l'implant peut être soutenu sur une paroi vasculaire,
(c) une partie perméable pour la région de la ramification de vaisseaux, et
(d) une partie distale, dans laquelle l'implant est élargi radialement par rapport à la partie (b) et qui se destine à être placée dans l'anévrisme (A),
dans lequel est disposée, dans la région des parties (c) ou (d), une zone de séparation (T1, T2) qui est configurée de telle sorte qu'elle obture au moins en partie le col de l'anévrisme,
la partie distale (d) présente une multitude de boucles (12) reliées à la partie (c) et les boucles (12) forment par rapport à l'axe longitudinal de l'implant (1) un angle entre -45° et +175°, dans lequel un angle positif indique que les boucles (12) pointent vers l'extérieur radialement et un angle négatif indique que les boucles pointent vers l'intérieur radialement,
**caractérisé en ce**
**que** la zone de séparation présente une ou plusieurs membranes (16, 24), qui remplissent l'intérieur des boucles (12) et recouvrent des espaces intermédiaires entre les boucles (12).

2. Implant selon la revendication 1, **caractérisé en ce que** la ou les membranes (16, 24) sont tendues dans la région de la zone de séparation (T1, T2) de manière orthogonale par rapport à l'axe longitudinal de l'implant (1).

3. Implant selon l'une quelconque des revendications 1 à 2, **caractérisé en ce que** les membranes (16, 24) s'étendent dans le sens proximal et forment de préférence une forme de cône ou de pyramide.

4. Implant selon l'une quelconque des revendications 1 à 3, **caractérisé en ce que** les boucles (12) forment par rapport à l'axe longitudinal de l'implant (1) un angle entre +45° et +90°.

5. Implant selon l'une quelconque des revendications 1 à 4, **caractérisé en ce que** les boucles (12) présentent dans la partie distale (d) des œillets (17).

6. Implant selon l'une quelconque des revendications 1 à 5, **caractérisé en ce que** les boucles (12) présentent dans la partie distale (d) des ergots (18) sur l'extrémité distale.

7. Implant selon l'une quelconque des revendications 1 à 6, **caractérisé en ce que** les boucles (12) sont fixées par l'intermédiaire d'individuels, en particulier de respectivement un ou deux points de liaison sur la partie (c).

8. Implant selon l'une quelconque des revendications 1 à 7, **caractérisé en ce que** l'implant (1) présente du côté proximal de la partie (b) une partie (a), dans lequel la partie (a) est une partie proximale se rétrécissant, dans laquelle la structure à mailles (3, 4) est rassemblée en un ou plusieurs éléments de couplage (10), de préférence en fils de couplage.

9. Implant selon la revendication 8,
**caractérisé en ce que** les éléments de couplage (10) sont rassemblés de manière excentrée sur la périphérie de l'implant (1) dans sa forme expansée.

10. Implant selon la revendication 9,
**caractérisé en ce que** les éléments de couplage (10), de préférence les fils de couplage, forment par rapport à l'axe longitudinal de l'implant (1) un angle entre 0° et +60°, de préférence +10° jusqu'à + 30°, dans lequel un angle positif indique des éléments de couplage (10) qui pointent vers l'extérieur.

11. Implant selon l'une quelconque des revendications 1 à 10, **caractérisé en ce**
**que** la zone de séparation (T1, T2) présente des éléments de séparation composés de filaments (6) s'étendant de manière orthogonale par rapport à l'axe longitudinal de l'implant (1), de préférence sensiblement dans un plan.
